# EUROPEAN PATENT APPLICATION

(11) **EP 4 501 306 A1**
(43) Date of publication of application: **05.02.2025**
(21) Application number: 23775107.8
(22) Date of filing: 24.03.2023
(51) Int. Cl.: A61K 6/887, A61K 6/30, A61K 6/62

(54) **ADHESIVE COMPOSITION FOR STEREOLITHOGRAPHIC ARTICLE AND NON-STEREOLITHOGRAPHIC ARTICLE**

(30) Priority: 24.03.2022 JP 2022048700
(71) Applicant: Kuraray Noritake Dental Inc., Kurashiki-shi, Okayama 710-0801 (JP)
(72) Inventor: SUZUKI Kenji, Tainai-shi, Niigata 959-2653 (JP)
(74) Representative: D Young & Co LLP
(86) International application number: PCT/JP2023/011971
(87) International publication number: WO 2023/182514

(57) **Abstract**

The present invention provides an adhesive composition for stereolithographic articles and non-stereolithographic articles, possessing excellent adhesive properties to both objects fabricated by stereolithography and molded articles made by methods other than stereolithography. The present invention relates to an adhesive composition for stereolithographic articles and non-stereolithographic articles, comprising: a polyfunctional (meth)acrylic polymerizable monomer (A); and an organic solvent (B) having a normal boiling point of 120°C or less, and a viscosity at 25°C of 10 mPa·s or less, wherein the polyfunctional (meth)acrylic polymerizable monomer (A) comprises two or more polymerizable groups per molecule, and at least one selected from the group consisting of a urethane bond, an aromatic skeleton, and an amide bond, and the polymerizable groups of the polyfunctional (meth)acrylic polymerizable monomer (A) are (meth)acryloxy groups or (meth)acrylamide groups.

## Description

### TECHNICAL FIELD

The present invention relates to adhesive compositions capable of adhering to both stereolithographic articles and non-stereolithographic articles. Specifically, the invention relates to an adhesive composition for stereolithographic articles and non-stereolithographic articles, possessing excellent adhesive properties to not only objects fabricated by stereolithography but also molded articles (non-stereolithographic articles) made from materials such as acrylic resins (for example, PMMA) through fabrication methods other than stereolithography (hereinafter, also referred to as "non-stereolithography methods"), enabling the repairing or lining of both objects fabricated by stereolithography and molded articles (non-stereolithographic articles) made from materials such as acrylic resins through polymerization using methods other than stereolithography (for example, thermal polymerization, photopolymerization). The invention is particularly suited for lining or repairing denture base materials, dental occlusal splints, and appliances used for treating sleep disorders.

### BACKGROUND ART

Various proposals have been made concerning stereolithography, a method that produces three-dimensional objects through a repeated procedure whereby liquid photocurable resin is cured into a thin layer under controlled application of necessary amounts of light energy, and another layer of photocurable resin is cured on the cured layer under controlled application of light after supplying another portion of the liquid photocurable resin onto the previously formed cured layer.

Vat stereolithography is a technique typically used for optical fabrication of three-dimensional objects. In this technique, a liquid photocurable resin composition is placed in a vat, and a computer-controlled ultraviolet laser is selectively applied to the surface of the resin composition to cure it to a predetermined thickness, forming a cured layer with the desired pattern. Continuously, another cured layer is formed on the cured layer by applying an ultraviolet laser in the same manner to the liquid photocurable resin composition supplied onto the previously cured layer in an amount necessary to form a single layer. This layering process is repeated until it produces the final three-dimensional object. This technique has attracted great interest because it enables easy and precision production of the desired three-dimensional object in a relatively short time period, even when the product has a very complex shape.

Three-dimensional objects created through stereolithography are expanding their applications from mere concept models to test models, prototypes, and final products. The field of dental materials is thought to greatly benefit from stereolithography because denture bases, and mouthpiece-like appliances used for treating sleep disorders (for example, appliances for preventing bruxism, and appliances for treating sleep apnea) require shapes that vary from patient to patient, aside from being complex in shape.

Denture base materials are materials applied to the gum area when dentures are fitted following the loss of teeth. The demand for dentures has rapidly increased in recent years because of increasing ageing populations. Additionally, denture base materials often require lining (rebasing or relining as it is also called) or repairs due to changes such as deformation occurring in the material itself, or shifts in the patient's alveolar ridge. The lining and repairing of denture bases typically involve the application of a methyl methacrylate-based mixture of methacrylate monomer and methacrylate polymer after an optional surface treatment with a bonding agent containing a methacrylate polymer dissolved in an organic solvent such as ethyl acetate or methylene chloride. Practically, this method is the only approach for such lining or repairing procedures. The same technique is employed in the repair of mouthpiece-like appliances used for treating sleep disorders, for example, such as appliances for preventing bruxism, and appliances for treating sleep apnea.

With regard to the fabrication of denture bases themselves, the traditional mainstream method produces denture base materials through thermal polymerization from a mixture of methyl methacrylate (MMA) and polymethyl methacrylate (PMMA). Alternatively, denture base materials can also be produced by stereolithography. Stereolithography involves the instant light curing process, and requires the use of highly curable polymerizable compounds for denture base materials. The stereolithographical fabrication of denture base materials thus involves the use of crosslinkable monomers, or monomers that exhibit high cohesive strength. Additionally, it is impractical to use MMA because the structure of stereolithography device does not allow the use of volatile components.

Because of these issues related to materials, denture base materials fabricated by stereolithography have a high crosslinking density, preventing easy penetration of lining materials for denture bases or bonding agents for lining denture bases, posing difficulty for lining and repairs. Consequently, there is a demand for the development of bonding agents for denture bases fabricated by stereolithography.

A wide range of dental adhesive materials (adhesive compositions) are known, including those applied to the tooth structure, those applied to dental composite resins, and those applied to metal or ceramic dental prostheses. Dental adhesive materials intended for these applications, such as acidic monomers, silane coupling agents, and sulfur-containing monomers, exhibit excellent affinity and form chemical bonds with adherends such as the tooth structure. However, the adhesive materials used in these applications are fundamentally different from adhesive materials intended for denture base materials, dental occlusal splints, and appliances for sleep disorder treatment in terms of technology and the mechanism of exhibiting adhesive properties.

Against this backdrop, for example, Patent Literature 1 discloses examples of compositions with excellent adhesive properties, provided as mixtures of polymers and radical polymerizable monomers diluted with solvent, as a technique to reline denture bases.

### CITATION LIST

### Patent Literature

Patent Literature 1: JP 2008-214359 A

### SUMMARY OF INVENTION

### Technical Problem

However, Patent Literature 1 concerns lining of PMMA denture base materials, and does not include descriptions concerning highly crosslinked systems or stereolithographic articles.

Moreover, there is a challenge in clinical settings to distinguish between dentures created by stereolithography and those fabricated using traditional methods. This has led to difficulties in choosing a bonding agent. Consequently, there is a strong demand for bonding agents capable of bonding to both denture bases created by stereolithography and denture bases fabricated using traditional methods.

It is accordingly an object of the present invention to provide an adhesive composition for stereolithographic articles and non-stereolithographic articles, possessing excellent adhesive properties to both objects fabricated by stereolithography and molded articles made by methods other than stereolithography.

### Solution to Problem

The present invention includes the following.
[1] An adhesive composition for stereolithographic articles and non-stereolithographic articles, comprising:
   a polyfunctional (meth)acrylic polymerizable monomer (A); and
   an organic solvent (B) having a normal boiling point of 120°C or less, and a viscosity at 25°C of 10 mPa·s or less,
   wherein the polyfunctional (meth)acrylic polymerizable monomer (A) comprises two or more polymerizable groups per molecule, and at least one selected from the group consisting of a urethane bond, an aromatic skeleton, and an amide bond, and
   the polymerizable groups of the polyfunctional (meth)acrylic polymerizable monomer (A) are (meth)acryloxy groups or (meth)acrylamide groups.
[2] The adhesive composition for stereolithographic articles and non-stereolithographic articles according to [1], wherein the polyfunctional (meth)acrylic polymerizable monomer (A) comprises at least one selected from the group consisting of a urethanized (meth)acrylic acid ester compound (a-1), an aromatic (meth)acrylic acid ester compound (a-2) with no urethane bond, and a polymerizable monomer (a-3) containing (meth)acrylamide.
[3] The adhesive composition for stereolithographic articles and non-stereolithographic articles according to [2], wherein the polyfunctional (meth)acrylic polymerizable monomer (A) comprises the urethanized (meth)acrylic acid ester compound (a-1).
[4] The adhesive composition for stereolithographic articles and non-stereolithographic articles according to [2], wherein the polyfunctional (meth)acrylic polymerizable monomer (A) comprises the urethanized (meth)acrylic acid ester compound (a-1), and the urethanized (meth)acrylic acid ester compound (a-1) is a compound with no polymer structure.
[5] The adhesive composition for stereolithographic articles and non-stereolithographic articles according to [4], wherein the urethanized (meth)acrylic acid ester compound (a-1) comprises at least one selected from the group consisting of 2,2,4-trimethylhexamethylenebis(2-carbamoyloxyethyl)dimethacrylate, and N,N'-(2,2,4-trimethylhexamethylene)bis[2-(aminocarboxy)propane-1,3-diol]tetramethacry late.
[6] The adhesive composition for stereolithographic articles and non-stereolithographic articles according to [2], wherein the polyfunctional (meth)acrylic polymerizable monomer (A) comprises the aromatic (meth)acrylic acid ester compound (a-2) with no urethane bond, and the aromatic (meth)acrylic acid ester compound (a-2) with no urethane bond further comprises a hydroxyl group.
[7] The adhesive composition for stereolithographic articles and non-stereolithographic articles according to [2], wherein the polyfunctional (meth)acrylic polymerizable monomer (A) comprises the polymerizable monomer (a-3) containing (meth)acrylamide, and the polymerizable monomer (a-3) containing (meth)acrylamide comprises at least one selected from the group consisting of a primary amide bond and a secondary amide bond.
[8] The adhesive composition for stereolithographic articles and non-stereolithographic articles according to any one of [1] to [7], wherein the organic solvent (B) comprises at least one selected from the group consisting of a halogenated hydrocarbon solvent, an ether solvent, an ester solvent, a ketone solvent, a non-aromatic hydrocarbon solvent, and an alcohol solvent.
[9] The adhesive composition for stereolithographic articles and non-stereolithographic articles according to any one of [1] to [8], wherein the organic solvent (B) comprises at least one selected from the group consisting of ethyl acetate, methylene chloride, and acetone.
[10] The adhesive composition for stereolithographic articles and non-stereolithographic articles according to any one of [1] to [9], wherein the content of the organic solvent (B) is 40 to 85 mass%.
[11] The adhesive composition for stereolithographic articles and non-stereolithographic articles according to any one of [1] to [10], which comprises 1 mass% or less of a polymer component with a weight-average molecular weight of 1,000 or more.
[12] The adhesive composition for stereolithographic articles and non-stereolithographic articles according to any one of [1] to [11], which further comprises a polymerization initiator (C).
[13] The adhesive composition for stereolithographic articles and non-stereolithographic articles according to any one of [1] to [12], which further comprises a polymerization accelerator (D).
[14] A bonding agent for dental oral appliances, comprising an adhesive composition for stereolithographic articles and non-stereolithographic articles of any one of [1] to [13].
[15] A bonding agent for denture bases, comprising an adhesive composition for stereolithographic articles and non-stereolithographic articles of any one of [1] to [13].
[16] A bonding agent for dental occlusal splints, comprising an adhesive composition for stereolithographic articles and non-stereolithographic articles of any one of [1] to [13].
[17] A bonding agent for therapeutic appliances for sleep apnea, comprising an adhesive composition for stereolithographic articles and non-stereolithographic articles of any one of [1] to [13].
[18] A method for lining or repairing stereolithographic articles and non-stereolithographic articles fabricated by stereolithography or methods other than stereolithography, using an adhesive composition for stereolithographic articles and non-stereolithographic articles of any one of [1] to [13].
[19] An adhesive composition comprising:
   a polyfunctional (meth)acrylic polymerizable monomer (A); and
   an organic solvent (B) having a normal boiling point of 120°C or less, and a viscosity at 25°C of 10 mPa·s or less,
   wherein the polyfunctional (meth)acrylic polymerizable monomer (A) comprises two or more polymerizable groups per molecule, and at least one selected from the group consisting of a urethane bond, an aromatic skeleton, and an amide bond,
   the polymerizable groups of the polyfunctional (meth)acrylic polymerizable monomer (A) are (meth)acryloxy groups or (meth)acrylamide groups, and
   the polyfunctional (meth)acrylic polymerizable monomer (A) comprises a urethanized (meth)acrylic acid ester compound (a-1).

### Advantageous Effects of Invention

According to the present invention, an adhesive composition for stereolithographic articles and non-stereolithographic articles can be provided that possesses excellent adhesive properties to both objects fabricated by stereolithography and molded articles made by methods other than stereolithography.

An adhesive composition for stereolithographic articles and non-stereolithographic articles of the present invention can easily penetrate objects fabricated by stereolithography involving high crosslinking density (hereinafter, also referred to as "stereolithographic articles"), as well as molded articles made by methods other than stereolithography (hereinafter, also referred to as "non-stereolithographic articles"), enabling the repair or lining of both stereolithographic articles and non-stereolithographic articles. An adhesive composition for stereolithographic articles and non-stereolithographic articles of the present invention is particularly suited for lining or repairing denture base materials, dental occlusal splints, and appliances used for treating sleep disorders.

Specifically, in dental treatment, it is no longer necessary to determine whether the denture base material is a stereolithographic article or non-stereolithographic article in lining denture bases, enabling use regardless of the denture base material.

Traditional adhesive compositions (for example, those primarily composed of (meth)acrylate polymers) may lack adhesive properties depending on the denture base material, necessitating a process to assess the denture base material. If the denture base material cannot be visually distinguished with certainty, there remains a risk of detachment when repairing or lining the denture base.

In contrast to such traditional adhesive compositions, an adhesive composition of the present invention possesses excellent adhesive properties to both stereolithographic articles and non-stereolithographic articles, regardless of the denture base material. This eliminates the need to assess the denture base material, and removes the risk of detachment, offering significant advantages in clinical dental practice.

### DESCRIPTION OF EMBODIMENTS

An adhesive composition for stereolithographic articles and non-stereolithographic articles of the present invention comprises a polyfunctional (meth)acrylic polymerizable monomer (A); and an organic solvent (B) having a normal boiling point of 120°C or less, and a viscosity at 25°C of 10 mPa·s or less.

In this specification, the methods other than stereolithography (non-stereolithography methods) are not particularly limited, as long as it is not stereolithography. Examples include photopolymerization, thermal polymerization, and chemical polymerization. Examples of thermal polymerization include methods involving thermal polymerization of a mixture of methyl methacrylate (MMA) and polymethyl methacrylate (PMMA). The thermal polymerization process is not particularly limited, and may be conducted using known methods and devices, including commercially available products (such as automatic polymerizers).

A certain embodiment is, for example, an adhesive composition for stereolithographic articles and non-stereolithographic articles where the non-stereolithographic article is a polymer (molded article) molded into a predetermined shape through thermal polymerization in a mold.

Examples of photopolymerization include methods that achieve photopolymerization by exposing the composition to light for longer periods than stereolithography, using techniques other than stereolithography.

Another embodiment is, for example, an adhesive composition for stereolithographic articles and non-stereolithographic articles where the non-stereolithographic article is a polymer (molded article) molded into a predetermined shape through photopolymerization in a mold.

In this specification, the upper limits and lower limits of numeric ranges (for example, ranges of contents of components, ranges of values calculated from components, and ranges of physical properties) can be appropriately combined. In this specification, the numeric values represented by symbols in the formulae can also be combined as appropriate. In this specification, the term "(meth)acryl" is intended to be inclusive of both methacryl and acryl, and the same applies to similar expressions such as "(meth)acryloyl" and "(meth)acrylate". The term "(meth)acrylic" is intended to encompass (meth)acrylic acid esters and (meth)acrylamides.

### [Polyfunctional (Meth)Acrylic Polymerizable Monomer (A)]

In an adhesive composition for stereolithographic articles and non-stereolithographic articles of the present invention, the polyfunctional (meth)acrylic polymerizable monomer (A) is used to impart adhesive properties to stereolithographic articles.

The curability of the composition greatly influences how it exhibits adhesive properties to stereolithographic articles. The polyfunctional (meth)acrylic polymerizable monomer (A), with organic solvent (B), can impart high curability to the composition while maintaining the adhesive properties of organic solvent (B) to non-stereolithographic articles. The polyfunctional (meth)acrylic polymerizable monomer (A) is required to comprise two or more (meth)acrylic polymerizable groups per molecule. Here, "(meth)acrylic polymerizable group" refers to (meth)acryloxy group or (meth)acrylamide group. The polyfunctional (meth)acrylic polymerizable monomer (A) may have three or more (meth)acrylic polymerizable groups per molecule, or four or more, five or more, or six or more (meth)acrylic polymerizable groups per molecule. In certain embodiments, the polyfunctional (meth)acrylic polymerizable monomer (A) may have 2 to 6, 2 to 5, or 2 to 4 (meth)acrylic polymerizable groups per molecule.

The polyfunctional (meth)acrylic polymerizable monomer (A) comprises at least one selected from the group consisting of a urethane bond, an aromatic skeleton, and an amide bond. It is speculated that the dielectric effect of urethane bonds, aromatic skeletons, or amide bonds not only promotes curing but form chemical bonds such as hydrogen bonds and π-electron interactions. The polyfunctional (meth)acrylic polymerizable monomer (A) may be used alone, or two or more thereof may be used in combination.

In view of producing notably high curability and achieving high adhesive properties, the polyfunctional (meth)acrylic polymerizable monomer (A) preferably comprises at least one selected from the group consisting of a urethanized (meth)acrylic acid ester compound (a-1), an aromatic (meth)acrylic acid ester compound (a-2) with no urethane bond, and a polymerizable monomer (a-3) containing (meth)acrylamide. In this specification, when the polyfunctional (meth)acrylic polymerizable monomer (A) comprises even one (meth)acrylamide group, it falls under the category of polymerizable monomer (a-3) containing (meth)acrylamide.

A certain preferred embodiment is, for example, an adhesive composition for stereolithographic articles and non-stereolithographic articles in which the polyfunctional (meth)acrylic polymerizable monomer (A) comprises a urethanized (meth)acrylic acid ester compound (a-1).

Another certain preferred embodiment is, for example, an adhesive composition for stereolithographic articles and non-stereolithographic articles in which the polyfunctional (meth)acrylic polymerizable monomer (A) comprises a urethanized (meth)acrylic acid ester compound (a-1), and the urethanized (meth)acrylic acid ester compound (a-1) is a compound with no polymer structure.

When the polyfunctional (meth)acrylic polymerizable monomer (A) comprises an aromatic (meth)acrylic acid ester compound (a-2) with no urethane bond, it is preferable that the aromatic (meth)acrylic acid ester compound (a-2) with no urethane bond further comprise a hydroxyl group. The dielectric effect of hydroxyl groups appears to not only promote curing to a greater extent but enable the formation of chemical bonds such as hydrogen bonds and π-electron interactions.

When the polyfunctional (meth)acrylic polymerizable monomer (A) comprises a polymerizable monomer (a-3) containing (meth)acrylamide, the polymerizable monomer (a-3) containing (meth)acrylamide preferably comprises at least one selected from the group consisting of a primary amide bond and a secondary amide bond. It is speculated that these compounds, with the dielectric effect of primary amide bonds and secondary amide bonds, not only promote curing but also enable the formation of chemical bonds such as hydrogen bonds and π-electron interactions.

### [Urethanized (Meth)Acrylic Acid Ester Compound (a-1)]

The urethanized (meth)acrylic acid ester compound (a-1) can be easily synthesized, for example, through an addition reaction between a compound containing an isocyanate with an alkylene skeleton or phenylene skeleton, and a (meth)acrylate compound having a hydroxyl groups (-OH). In view of easier application due to low viscosity and from the perspective of facilitating penetration into stereolithographic articles, the weight-average molecular weight is preferably less than 1,000.

Preferably, the alkylene skeleton is, for example, a compound having an alkylene group.

The alkylene group constituting the alkylene skeleton has preferably 1 to 30 carbon atoms, more preferably 2 to 20 carbon atoms, even more preferably 3 to 18 carbon atoms, particularly preferably 4 to 15 carbon atoms.

The alkylene group may be linear or branched.

Examples of the alkylene group include methylene groups, ethylene groups, n-propylene groups, isopropylene groups, trimethylene groups, tetramethylene groups, pentamethylene groups, hexamethylene groups, heptamethylene groups, octamethylene groups, tetradecylene groups, hexadecylene groups, octadecylene groups, and eicosanylene groups.

When the urethanized (meth)acrylic acid ester compound (a-1) contains a polymer structure, the urethanized (meth)acrylic acid ester compound (a-1) can be easily synthesized through an addition reaction between a polyol having a polymer skeleton, a compound having an isocyanate group, and a (meth)acrylate compound having a hydroxyl group. The urethanized (meth)acrylic acid ester compound (a-1) can also be synthesized with ease by allowing lactone or alkylene oxide to undergo a ring-opening addition reaction with a (meth)acrylate compound having a hydroxyl group, and causing the resulting compound with a terminal hydroxyl group to undergo an addition reaction with a compound having an isocyanate group.

When the urethanized (meth)acrylic acid ester compound (a-1) contains a polymer structure, the urethanized (meth)acrylic acid ester compound (a-1) is preferably a (meth)acrylate that, in addition to the urethane bond, has a structure (polymer skeleton) selected from the group consisting of a polyester, a polycarbonate, a polyurethane, a polyether, a poly-conjugated diene, and a hydrogenated poly-conjugated diene.

When the urethanized (meth)acrylic acid ester compound (a-1) does not contain a polymer structure, the molecular weight is employed because the concept of weight-average molecular weight does not apply to such compounds.

When the urethanized (meth)acrylic acid ester compound (a-1) has a polymer structure and a weight-average molecular weight of 1,500 or more, an adhesive composition for stereolithographic articles and non-stereolithographic articles of the present invention tends to be more viscous and show decreased penetration into stereolithographic articles. In view of adhesive properties to stereolithographic articles, the weight-average molecular weight is preferably less than 1,500, more preferably 1,100 or less, even more preferably less than 1,000, particularly preferably 750 or less, most preferably 500 or less. As used herein, "weight-average molecular weight" means a weight-average molecular weight in terms of polystyrene as determined by gel permeation chromatography (GPC).

Concerning the polymer skeleton of the polyol contained in the polymer skeleton, examples of the polyester include polymers of dicarboxylic acids (aromatic dicarboxylic acids such as phthalic acid and isophthalic acid, and unsaturated aliphatic dicarboxylic acids such as maleic acid) and aliphatic diols having 2 to 18 carbon atoms, polymers of dicarboxylic acids (saturated aliphatic dicarboxylic acids such as adipic acid and sebacic acid) and aliphatic diols having 2 to 18 carbon atoms, β-propiolactone polymers, γ-butyrolactone polymers, δ-valerolactone polymers, ε-caprolactone polymers, and copolymers of these. Preferred are polymers of dicarboxylic acids (aromatic dicarboxylic acids such as phthalic acid and isophthalic acid, and unsaturated aliphatic dicarboxylic acids such as maleic acid) and aliphatic diols having 2 to 12 carbon atoms, and polymers of dicarboxylic acids (saturated aliphatic dicarboxylic acids such as adipic acid and sebacic acid) and aliphatic diols having 2 to 12 carbon atoms.

Examples of the polycarbonate include polycarbonates derived from C2 to C18 aliphatic diols, polycarbonates derived from bisphenol A, and polycarbonates derived from C2 to C18 aliphatic diols and bisphenol A. Preferred are polycarbonates derived from C2 to C12 aliphatic diols, polycarbonates derived from bisphenol A, and polycarbonates derived from C2 to C12 aliphatic diols and bisphenol A.

Examples of the polyurethane include polymers of C2 to C18 aliphatic diols and C1 to C18 diisocyanates. Preferred are polymers of C2 to C12 aliphatic diols and C1 to C12 diisocyanates.

Examples of the polyether include polyethylene glycol, polypropylene glycol, polybutylene glycol, and poly(1-methylbutylene glycol).

Examples of the poly-conjugated diene and hydrogenated poly-conjugated diene include 1,4-polybutadiene, 1,2-polybutadiene, polyisoprene, poly(butadiene-isoprene), poly(butadiene-styrene), poly(isoprene-styrene), polyfarnesene, and hydrogenated products of these.

In view of superior mechanical strength and water resistance, preferred among the polyols having a polymer skeleton are polyols having a polyester structure, polyols having a polycarbonate structure, and polyols having a poly-conjugated diene structure.

In view of exhibiting even higher adhesive properties to both stereolithographic articles and non-stereolithographic articles, the urethanized (meth)acrylic acid ester compound (a-1) is preferably a compound with no polymer structure, such as polyesters, polycarbonates, polyurethanes, and polyethers. When the urethanized (meth)acrylic acid ester compound (a-1) has a polymer structure, the higher concentration of polar functional groups tends to lead to a decrease in adhesive properties in the presence of moisture such as in the oral cavity. In view of adhesive properties in the presence of moisture, the number of urethane bonds, in particular, is preferably three or less, more preferably two or less per molecule of urethanized (meth)acrylic acid ester compound (a-1).

Examples of the compound having an isocyanate group include methylene diisocyanate (MDI), hexamethylene diisocyanate (HDI), isophorone diisocyanate (IPDI), trimethylhexamethylene diisocyanate (TMHMDI), tricyclodecane diisocyanate (TCDDI), adamantane diisocyanate (ADI), tolylene diisocyanate (TDI), xylylene diisocyanate (XDI), and diphenylmethane diisocyanate (MDI).

In view of an adhesive composition for stereolithographic articles and non-stereolithographic articles of the present invention exhibiting superior adhesive properties to stereolithographic articles, preferred are HDI, IPDI, TMHMDI, and TCDDI, more preferably IPDI and TMHMDI.

Examples of the (meth)acrylate compound having a hydroxyl group include hydroxy (meth)acrylate compounds such as 2-hydroxyethyl (meth)acrylate, 2-hydroxypropyl (meth)acrylate, 2-hydroxybutyl (meth)acrylate, 6-hydroxyhexyl (meth)acrylate, 10-hydroxydecyl (meth)acrylate, 3-chloro-2-hydroxypropyl (meth)acrylate, 2-hydroxy-3-phenoxypropyl (meth)acrylate, glycerin mono(meth)acrylate, 2-hydroxy-3-acryloyloxypropyl (meth)acrylate, 2,2-b is[4-[3-(m eth)acryloyloxy-2-hydroxypropoxy]phenyl] propane, 1,2-bis[3-(meth)acryloyloxy-2-hydroxypropoxy]ethane, pentaerythritol tri(meth)acrylate, and tri or tetra(meth)acrylates of dipentaerythritol. These may be used alone, or two or more thereof may be used in combination.

In view of an adhesive composition for stereolithographic articles and non-stereolithographic articles of the present invention exhibiting superior adhesive properties to stereolithographic articles, preferred are 2-hydroxyethyl (meth)acrylate, 2-hydroxypropyl (meth)acrylate, and 2-hydroxy-3-acryloyloxypropyl (meth)acrylate, more preferably 2-hydroxyethyl (meth)acrylate.

The addition reaction between a polyol having an alkylene skeleton or polymer skeleton, a compound having an isocyanate group, and a (meth)acrylate compound having a hydroxyl group may follow known methods, and is not particularly limited.

Examples of the urethanized (meth)acrylic acid ester compound with no polymer structure include 2,2,4-trimethylhexamethylenebis(2-carbamoyloxyethyl)dimethacrylate (commonly known as UDMA), 2,4-tolylenebis(2-carbamoyloxyethyl)dimethacrylate, bishydroxyethyl methacrylate-isophorone diurethane, N,N'-(2,2,4-trimethylhexamethylene)bis[2-(aminocarboxy)propane-1,3-diol]tetramethacry late, hexamethylenebis{2-carbamoyloxy-3-phenoxypropyl}diacrylate, and 2,4-tolylenebis(2-carbamoyloxyethyl)hexaacrylate. These may be used alone, or two or more thereof may be used in combination. In view of adhesive properties to stereolithographic articles, preferred are 2,2,4-trimethylhexamethylenebis(2-carbamoyloxyethyl)dimethacrylate, and N,N'-(2,2,4-trimethylhexamethylene)bis[2-(aminocarboxy)propane-1,3-diol]tetramethacry late, more preferably 2,2, 4-trim ethyl hexam ethylenebis(2 -carbam oyloxyethyl)d i methacrylate.

When an adhesive composition for stereolithographic articles and non-stereolithographic articles of the present invention comprises a urethanized (meth)acrylic acid ester compound (a-1), the content of the urethanized (meth)acrylic acid ester compound (a-1) in an adhesive composition for stereolithographic articles and non-stereolithographic articles of the present invention is preferably 10 to 100 parts by mass in total 100 parts by mass of the polymerizable monomers. In view of even superior adhesive properties to stereolithographic articles, the content is more preferably 30 to 90 parts by mass, even more preferably 50 to 80 parts by mass.

In view of even superior bond strength to stereolithographic articles and non-stereolithographic articles, the total amount of urethanized (meth)acrylic acid ester compound (a-1) is preferably 5 to 95 mass%, more preferably 6 to 92 mass%, even more preferably 7 to 90 mass% of the total composition amount.

### [Aromatic (Meth)AcrylicAcid Ester Compound (a-2) with no Urethane Bond]

Examples of the aromatic (meth)acrylic acid ester compound (a-2) with no urethane bond include 2,2-bis((meth)acryloyloxyphenyl)propane, 2,2-bis[4-(3-acryloyloxy)-2-hydroxypropoxyphenyl]propane, 2,2-bis[4-(2-hydroxy-3-methacryloyloxypropoxy)phenyl]propane (commonly known as Bis-GMA), 2,2-bis[4-{2-(3-acryloyloxy-2-hydroxypropoxy)propyl}phenyl]propane, 2,2-bis[4-{2-(3-methacryloyloxy-2-hydroxypropoxy)propyl}phenyl]propane, 2,2-bis(4-(meth)acryloyloxyethoxyphenyl)propane, 2,2-bis(4-(meth)acryloyloxypolyethoxyphenyl)propane, 2,2-bis(4-(meth)acryloyloxydiethoxyphenyl)propane, 2,2-bis(4-(meth)acryloyloxytetraethoxyphenyl)propane, 2,2-bis(4-(meth)acryloyloxypentaethoxyphenyl)propane, 2,2-bis(4-(meth)acryloyloxydipropoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2-(4-(meth)acryloyloxydipropoxyphenyl)-2-(4-(meth)acryloyloxytriethoxyphenyl)propane, 2,2-bis(4-(meth)acryloyloxypropoxyphenyl)propane, 2,2-bis(4-(meth)acryloxyisopropoxyphenyl)propane, and 1,4-bis(2-(meth)acryloyloxyethyl)pyromellitate. These may be used alone, or two or more thereof may be used in combination.

In view of superior adhesive properties to stereolithographic articles, preferred among these are compounds containing a hydroxyl group, specifically, 2,2-bis[4-(3-acryloyloxy)-2-hydroxypropoxyphenyl]propane, 2,2-b is[4-(2-hydroxy-3-m ethacryloyloxypropoxy) phenyl] propane, 2,2-bis[4-{2-(3-acryloyloxy-2-hydroxypropoxy)propyl}phenyl]propane, and 2,2-bis[4-{2-(3-methacryloyloxy-2-hydroxypropoxy)propyl}phenyl]propane, more preferably 2,2-bis[4-(2-hydroxy-3-methacryloyloxypropoxy)phenyl]propane.

When an adhesive composition for stereolithographic articles and non-stereolithographic articles of the present invention comprises an aromatic (meth)acrylic acid ester compound (a-2) with no urethane bond, the content of the aromatic (meth)acrylic acid ester compound (a-2) with no urethane bond in an adhesive composition for stereolithographic articles and non-stereolithographic articles of the present invention is preferably 5 to 90 parts by mass in total 100 parts by mass of the polymerizable monomers. In view of even superior adhesive properties to stereolithographic articles, the content is more preferably 10 to 80 parts by mass, even more preferably 20 to 70 parts by mass.

In view of even superior bond strength to stereolithographic articles and non-stereolithographic articles, the total amount of the aromatic (meth)acrylic acid ester compound (a-2) with no urethane bond is preferably 4 to 92 mass%, more preferably 5 to 90 mass%, even more preferably 6 to 85 mass% of the total composition amount.

### [Polymerizable Monomer (a-3) Containing (Meth)Acrylamide]

The polymerizable monomer (a-3) containing (meth)acrylamide has one or more (meth)acrylamide groups, and may be, for example, a compound having one (meth)acrylamide group and one (meth)acryloxy group, a compound having two (meth)acrylamide groups, or a compound having two (meth)acrylamide groups and one (meth)acryloxy group.

Examples of the polymerizable monomer (a-3) containing (meth)acrylamide include N,N'-ethylenebis(meth)acrylamide, N,N'-propylenelenebis(meth)acrylamide, N,N'-butylenebis(meth)acrylamide, N,N'-hexamethylenebis(meth)acrylamide, 2-(meth)acryloyloxyethyl(meth)acrylamide, 2-(meth)acryloyloxypropyl(meth)acrylamide, 2-(meth)acryloyloxybutyl(meth)acrylamide, and 2-(meth)acryloyloxyhexyl(meth)acrylamide. The polymerizable monomer (a-3) containing (meth)acrylamide may be an asymmetric acrylamide methacrylic acid ester compound represented by the following general formula (1).

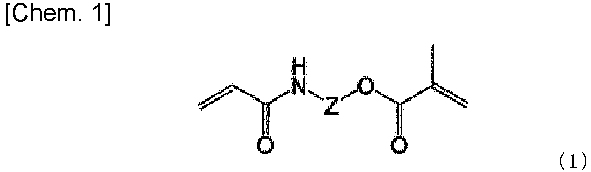

In the formula, Z is an optionally substituted C₁ to C₈ linear or branched aliphatic group or an optionally substituted aromatic group, and the aliphatic group may be interrupted with at least one binding group selected from the group consisting of -O-, -S-, -CO-, -CO-O-, -O-CO-, -NR¹-, -CO-NR¹-, -NR¹-CO-, -CO-O-NR¹-, -O-CO-NR¹-, and -NR¹-CO-NR¹-. R¹ represents a hydrogen atom, or an optionally substituted C₁ to C₈ linear or branched aliphatic group.

Z is a moiety adjusting the polarity of the asymmetric acrylamide-methacrylic acid ester compound.

The optionally substituted C₁ to C₈ aliphatic group represented by Z may be either a saturated aliphatic group (an alkylene group, or cycloalkylene group, for example, such as a 1 ,4-cyclohexylene group), or an unsaturated aliphatic group (an alkenylene group, an alkynylene group). In view of availability, ease of production, and chemical stability, preferred are saturated aliphatic groups (alkylene groups). In view of adhesive properties to stereolithographic articles and polymerization curability, Z is preferably an optionally substituted linear or branched C₁ to C₄ aliphatic group, more preferably an optionally substituted linear or branched C₂ to C₄ aliphatic group. Preferred as aliphatic groups are alkylene groups. Examples of the C₁ to C₈ alkylene group include methylene groups, ethylene groups, n-propylene groups, isopropylene groups, and n-butylene groups.

Examples of the optionally substituted aromatic group represented by Z include arylene groups, and aromatic heterocyclic groups. The aromatic group is more preferably an arylene group than an aromatic heterocyclic group. The hetero ring on the aromatic heterocyclic groups is typically unsaturated. The aromatic hetero ring is preferably a five-membered ring or a six-membered ring.

The arylene groups are preferably phenylene groups, for example. Examples of the hetero ring on the aromatic heterocyclic groups include a furan ring, a thiophene ring, a pyrrole ring, an oxazole ring, an isoxazole ring, a thiazole ring, an isothiazole ring, an imidazole ring, a pyrazole ring, a furazan ring, a triazole ring, a pyran ring, a pyridine ring, a pyridazine ring, a pyrimidine ring, a pyrazine ring, and a 1 ,3,5-triazine ring. Particularly preferred among these aromatic groups are phenylene groups.

The aliphatic group represented by R¹ may be either a saturated aliphatic group (an alkyl group) or an unsaturated aliphatic group (an alkenyl group, an alkynyl group). In view of availability, ease of production, and chemical stability, preferred are saturated aliphatic groups (alkyl groups).

Examples of the alkyl groups in R¹ include C₁ to C₆ linear or branched alkyl groups. Preferred are C₁ to C₃ linear alkyl groups, more preferably methyl groups or ethyl groups, even more preferably methyl groups.

The asymmetric acrylamide methacrylic acid ester compound is not particularly limited to specific examples, and includes the following.

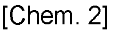

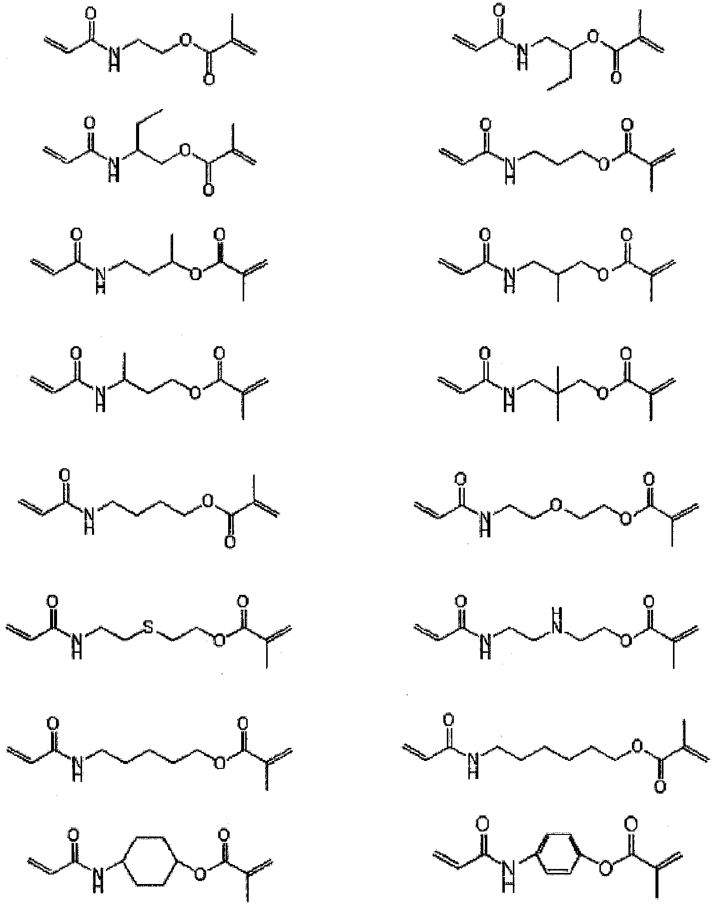

In view of superior adhesive properties to stereolithographic articles, preferred among these is 2-(meth)acryloyloxyethyl(meth)acrylamide, more preferably 2-methacryloyloxyethylacrylam ide.

The content of the polymerizable monomer (a-3) containing (meth)acrylamide in an adhesive composition for stereolithographic articles and non-stereolithographic articles of the present invention is preferably 10 to 100 parts by mass in total 100 parts by mass of the polymerizable monomers. In view of even superior bond strength to stereolithographic articles, the content is more preferably 30 to 90 parts by mass, even more preferably 50 to 80 parts by mass.

In view of even superior bond strength to stereolithographic articles and non-stereolithographic articles, the total content of polymerizable monomer (a-3) containing (meth)acrylamide is preferably 5 to 95 mass%, more preferably 6 to 92 mass%, even more preferably 7 to 90 mass% of the total composition amount.

The total amount of the polyfunctional (meth)acrylic polymerizable monomer (A) in an adhesive composition for stereolithographic articles and non-stereolithographic articles of the present invention is preferably 10 to 100 parts by mass in total 100 parts by mass of the polymerizable monomers. In view of even superior adhesive properties to stereolithographic articles, the total amount of polyfunctional (meth)acrylic polymerizable monomer (A) is more preferably 30 to 90 parts by mass, even more preferably 50 to 80 parts by mass.

In a preferred aspect, the total content of the urethanized (meth)acrylic acid ester compound (a-1), aromatic (meth)acrylic acid ester compound (a-2) with no urethane bond, and polymerizable monomer (a-3) containing (meth)acrylamide in the (meth)acrylic polymerizable monomer (A) is preferably 80 parts or more by mass, more preferably 90 parts or more by mass, even more preferably 95 parts or more by mass in total 100 parts by mass of the polymerizable monomer.

The components (a-1), (a-2), and (a-3) may be used alone, or two or more thereof may be used in combination.

In view of even superior bond strength to stereolithographic articles and non-stereolithographic articles, the total amount of polyfunctional (meth)acrylic polymerizable monomer (A) is preferably 5 to 95 mass%, more preferably 6 to 92 mass%, even more preferably 7 to 90 mass% of the total composition amount.

An adhesive composition for stereolithographic articles and non-stereolithographic articles of the present invention may comprise polyfunctional (meth)acrylic polymerizable monomers other than polyfunctional (meth)acrylic polymerizable monomer (A) (hereinafter, such monomers are also referred to as "additional polyfunctional (meth)acrylic polymerizable monomers").

Examples of the additional polyfunctional (meth)acrylic polymerizable monomers include:
bifunctional (meth)acrylic polymerizable monomers such as glycerol di(meth)acrylate, ethylene glycol di(meth)acrylate, diethylene glycol di(meth)acrylate, triethylene glycol di(meth)acrylate, propylene glycol di(meth)acrylate, butylene glycol di(meth)acrylate, neopentyl glycol di(meth)acrylate, polyethylene glycol di(meth)acrylate, 1,3-butanediol di(meth)acrylate, 1,4-butanediol di(meth)acrylate, 1,6-hexanediol di(meth)acrylate, 2-ethyl-1,6-hexanediol di(meth)acrylate, 1,9-nonanediol di(meth)acrylate, 1,10-decanediol di(meth)acrylate, and 1,2-bis(3-methacryloyloxy-2-hydroxypropoxy)ethane; and
tri- and higher-functional (meth)acrylic polymerizable monomers such as trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, and dipentaerythritol penta(meth)acrylate.

In view of superior adhesive properties to stereolithographic articles, preferred among these are bifunctional (meth)acrylic polymerizable monomers, more preferably glycerol di(meth)acrylate, and 1,2-bis(3-methacryloyloxy-2-hydroxypropoxy)ethane. These may be used alone, or two or more thereof may be used in combination.

When an adhesive composition for stereolithographic articles and non-stereolithographic articles of the present invention comprises additional polyfunctional (meth)acrylic polymerizable monomers, the content of the additional polyfunctional (meth)acrylic polymerizable monomers in an adhesive composition for stereolithographic articles and non-stereolithographic articles of the present invention is preferably 40 parts or less by mass, more preferably 20 parts or less by mass, even more preferably 10 parts or less by mass in total 100 parts by mass of the polymerizable monomers.

In certain embodiments where an adhesive composition for stereolithographic articles and non-stereolithographic articles of the present invention comprises additional polyfunctional (meth)acrylic polymerizable monomers, and the content of organic solvent (B) is 35 mass% or more and 95 mass% or less, it is preferable that the content of the additional polyfunctional (meth)acrylic polymerizable monomers be 15 parts or more by mass and 80 parts or less by mass, more preferably 20 parts or more by mass and 75 parts or less by mass, even more preferably 25 parts or more by mass and 70 parts or less by mass in total 100 parts by mass of the polymerizable monomers.

In such embodiments, the content of polyfunctional (meth)acrylic polymerizable monomer (A) is preferably 20 parts or more by mass and 85 parts or less by mass, more preferably 25 parts or more by mass and 80 parts or less by mass, even more preferably 30 parts or more by mass and 75 parts or less by mass in total 100 parts by mass of the polymerizable monomers.

An adhesive composition for stereolithographic articles and non-stereolithographic articles of the present invention may comprise a monofunctional (meth)acrylic polymerizable monomer for purposes such as adjusting viscosity, provided that it does not hinder the intent and purpose of the present invention.

Examples of the monofunctional (meth)acrylic polymerizable monomer include monofunctional (meth)acrylic polymerizable monomers with an aromatic ring, alicyclic (meth)acrylic polymerizable monomers, cyclic (meth)acrylic polymerizable monomers with a nitrogen atom, chain (meth)acrylic polymerizable monomers, cyclic (meth)acrylamide compounds, and chain (meth)acrylamide compounds. In view of high curability and weaker interference with adhesive properties, preferred for use are cyclic (meth)acrylic polymerizable monomers with a nitrogen atom, cyclic (meth)acrylamide compounds, and chain (meth)acrylamide compounds.

It should be noted that some monofunctional (meth)acrylic polymerizable monomers fall under the definition of the organic solvent (B) described later. In this specification, such monomers will be regarded as organic solvent (B), rather than monofunctional (meth)acrylic polymerizable monomers. For example, methyl methacrylate is an example of a monomer that falls under the definition of organic solvent (B). As an example, when an adhesive composition for stereolithographic articles and non-stereolithographic articles of the present invention comprises methyl methacrylate, it will be regarded as containing organic solvent (B), rather than a monofunctional (meth)acrylic polymerizable monomer.

Examples of the (meth)acrylic polymerizable monomers with an aromatic ring include o-phenylphenol (meth)acrylate, m-phenylphenol (meth)acrylate, p-phenylphenol (meth)acrylate, methoxylated-o-phenylphenol (meth)acrylate, methoxylated-m-phenylphenol (meth)acrylate, methoxylated-p-phenylphenol (meth)acrylate, ethoxylated-o-phenylphenol (meth)acrylate, ethoxylated-m-phenylphenol (meth)acrylate, ethoxylated-p-phenylphenol (meth)acrylate, propoxylated-o-phenylphenol (meth)acrylate, propoxylated-m-phenylphenol (meth)acrylate, propoxylated-p-phenylphenol (meth)acrylate, butoxylated-o-phenylphenol (meth)acrylate, butoxylated-m-phenylphenol (meth)acrylate, butoxylated-p-phenylphenol (meth)acrylate, o-phenoxybenzyl (meth)acrylate, m-phenoxybenzyl (meth)acrylate, p-phenoxybenzyl (meth)acrylate, 2-(o-phenoxyphenyl)ethyl (meth)acrylate, 2-(m-phenoxyphenyl)ethyl (meth)acrylate, 2-(p-phenoxyphenyl)ethyl (meth)acrylate, 3-(o-phenoxyphenyl)propyl (meth)acrylate, 3-(m-phenoxyphenyl)propyl (meth)acrylate, 3-(p-phenoxyphenyl)propyl (meth)acrylate, 4-(o-phenoxyphenyl)butyl (meth)acrylate, 4-(m-phenoxyphenyl)butyl (meth)acrylate, 4-(p-phenoxyphenyl)butyl (meth)acrylate, phenyl (meth)acrylate, 4-biphenylyl (meth)acrylate, 1-naphthyl (meth)acrylate, 2-naphthyl (meth)acrylate, anthryl (meth)acrylate, (meth)acrylic acid-o-2-propenylphenyl, benzhydrol (meth)acrylate, cumylphenol (meth)acrylate, fluorenyl (meth)acrylate, and fluorenylmethyl (meth)acrylate.

Examples of the alicyclic (meth)acrylic polymerizable monomers include 2-(1-adamantyl)propyl (meth)acrylate, 2-methyladamantyl-2-yl (meth)acrylate, 2-ethyladamantyl-2-yl (meth)acrylate, 2-n-propyladamantyl-2-yl (meth)acrylate, 2-isopropyladamantyl-2-yl (meth)acrylate, 1-(adamantan-1-yl)-1-methylethyl (meth)acrylate, 1-(adamantan-1-yl)-1-ethylethyl (meth)acrylate, 1-(adamantan-1-yl)-1-methylpropyl (meth)acrylate, and 1-(adamantan-1-yl)-1-ethylpropyl (meth)acrylate.

Examples of the cyclic (meth)acrylic polymerizable monomers with a nitrogen atom include pentamethylpiperidinyl (meth)acrylate, tetramethylpiperidinyl (meth)acrylate, and 4-(pyrimidin-2-yl)piperazin-1-yl (meth)acrylate.

Examples of the chain (meth)acrylic polymerizable monomers include 2-ethylhexyl (meth)acrylate, decyl (meth)acrylate, undecyl (meth)acrylate, lauryl (meth)acrylate, tridecyl (meth)acrylate, tetradecyl (meth)acrylate, pentadecyl (meth)acrylate, cetyl (meth)acrylate, palmitoleyl (meth)acrylate, heptadecyl (meth)acrylate, oleyl (meth)acrylate, stearyl (meth)acrylate, isostearyl (meth)acrylate, 4-hydroxybutyl (meth)acrylate, 6-hydroxyhexyl (meth)acrylate, 10-hydroxydecyl (meth)acrylate, glycerol mono(meth)acrylate, erythritol mono(meth)acrylate, and isobornylcyclohexyl (meth)acrylate.

Examples of the cyclic (meth)acrylamide compounds include N-(meth)acryloylmorpholine, N-(meth)acryloylpyrrolidine, N-(meth)acryloylpiperidine, N-(meth)acryloyl-2-methylpiperidine, and N-(meth)acryloyl-2,2,6,6-tetramethylpiperidine.

Examples of the chain (meth)acrylamide compounds include N,N-dimethyl(meth)acrylamide, N,N-diethyl(meth)acrylamide, N,N-di-n-propyl(meth)acrylamide, N-isopropyl(meth)acrylamide, N,N-di-n-butyl(meth)acrylamide, N,N-di-n-hexyl(meth)acrylamide, N,N-di-n-octyl(meth)acrylamide, N,N-di-2-ethylhexyl(meth)acrylamide, N-(2-hydroxyethyl)(meth)acrylamide, and N,N,N-bis(2-hydroxyethyl)(meth)acrylamide.

In view of an adhesive composition for stereolithographic articles and non-stereolithographic articles of the present invention exhibiting superior adhesive properties, examples of the monofunctional (meth)acrylic polymerizable monomers (excluding those classified as organic solvent (B)) include pentamethylpiperidinyl (meth)acrylate, tetramethylpiperidinyl (meth)acrylate, N-(meth)acryloylmorpholine, N,N-dimethyl(meth)acrylamide, and N,N-diethyl(meth)acrylamide.

The content of the monofunctional (meth)acrylic polymerizable monomers in an adhesive composition for stereolithographic articles and non-stereolithographic articles of the present invention is preferably 100 parts or less by mass in total 100 parts by mass of the polymerizable monomers. In view of even superior adhesive properties to stereolithographic articles, the content is more preferably 50 parts or less by mass, even more preferably 10 parts or less by mass.

The polymerizable monomers contained in an adhesive composition for stereolithographic articles and non-stereolithographic articles of the present invention may consist essentially of polyfunctional (meth)acrylic polymerizable monomer (A). The polymerizable monomers consisting essentially of polyfunctional (meth)acrylic polymerizable monomer (A) means that the content of polymerizable monomers other than polyfunctional (meth)acrylic polymerizable monomer (A) is less than 10.0 parts by mass, preferably less than 5.0 parts by mass, more preferably less than 1.0 part by mass, even more preferably less than 0.1 parts by mass, particularly preferably less than 0.01 parts by mass in total 100 parts by mass of the polymerizable monomers contained in the adhesive composition for stereolithographic articles and non-stereolithographic articles.

### [Organic Solvent (B)]

In an adhesive composition for stereolithographic articles and non-stereolithographic articles of the present invention, it is required to incorporate organic solvent (B) to impart adhesive properties to molded articles made from materials such as acrylic resins (for example, PMMA) through fabrication methods other than stereolithography (for example, thermal polymerization, photopolymerization) while maintaining the adhesive properties of polyfunctional (meth)acrylic polymerizable monomer (A) to stereolithographic articles. The organic solvent (B) may be used alone, or two or more thereof may be used in combination.

The organic solvent (B) has a normal boiling point of 120°C or less, and a viscosity at 25°C of 10 mPa·s or less. It is particularly preferable that the organic solvent (B) have the property to dissolve or swell non-stereolithographic articles (preferably, molded articles fabricated by non-stereolithography methods using acrylic resins such as PMMA) because it allows for easier penetration into non-stereolithographic articles. With this property to dissolve or swell non-stereolithographic articles, it is possible to impart adhesive properties to non-stereolithographic articles.

The organic solvent (B) has a normal boiling point of 120°C or less, more preferably 100°C or less, even more preferably 80°C or less. The organic solvent (B) has a viscosity at 25°C of 10 mPa·s or less, more preferably 5 mPa·s or less, even more preferably 2.5 mPa·s or less. The normal boiling point of organic solvent (B) is a measured value by atmospheric distillation. For compounds where the normal boiling point cannot be observed, it is determined by converting the boiling point at reduced pressure, measured by vacuum distillation, into an equivalent normal boiling point using a boiling point conversion table (Science of Petroleum, Vol.II. p.1281 (1938)).

Examples of the organic solvent (B) include:
non-aromatic hydrocarbon solvents, for example, alkanes (such as n-pentane, n-hexane, and n-heptane), cyclohexane, and methylcyclohexane;
aromatic hydrocarbon solvents, for example, benzene and toluene;
halogenated hydrocarbon solvents, for example, methylene chloride (dichloromethane), dichloroethane, chloroform, and carbon tetrachloride;
alcohol solvents, for example, ethanol, n-propanol, isopropanol, t-butanol, 1-butanol, 2-butanol, hexafluoroisopropanol, and allyl alcohol;
ether solvents, for example, n-propyl ether, isopropyl ether, diethyl ether, diisopropyl ether, methyl t-butyl ether, 1,2-dimethoxyethane, 1,1-dimethoxycyclohexane, dioxane, and tetrahydrofuran;
ester solvents, for example, carboxylic acid esters (such as ethyl formate, methyl acetate, ethyl acetate, isopropyl acetate, methyl propionate, and ethyl propionate), 3-methoxy-3-methylbutyl acetate, methyl methacrylate, dimethyl carbonate, and α-acetyl-γ-butyrolactone;
ketone solvents, for example, acetone, methyl ethyl ketone, and methyl isobutyl ketone;
carboxylic acid solvents, for example, acetic acid, and trifluoroacetic acid; and
nitrogen-containing solvents, for example, nitrile solvents such as acetonitrile, and nitro solvents such as nitromethane).

In view of superior adhesive properties to non-stereolithographic article (for example, acrylic resins) and easier evaporation after application, preferred among these are halogenated hydrocarbon solvents, ether solvents, ester solvents, ketone solvents, non-aromatic hydrocarbon solvents, and alcohol solvents, more preferably ethyl acetate, methyl methacrylate, methylene chloride, acetone, methyl ethyl ketone, ethanol, isopropanol, t-butanol, tetrahydrofuran, n-hexane, cyclohexane, and toluene, even more preferably ethyl acetate, methylene chloride, and acetone. In view of safety, ethyl acetate is particularly preferred.

The content of the organic solvent (B) in an adhesive composition for stereolithographic articles and non-stereolithographic articles of the present invention is preferably 10 to 10,000 parts by mass in total 100 parts by mass of the polymerizable monomers. In view of even superior bond strength to stereolithographic articles and non-stereolithographic articles, the content is more preferably 20 to 2,000 parts by mass, even more preferably 10 to 1,000 parts by mass. In view of even superior bond strength to stereolithographic articles and non-stereolithographic articles, the content of organic solvent (B) is preferably 5 to 95 mass%, more preferably 8 to 94 mass%, even more preferably 10 to 92 mass% of the total composition amount.

The content of organic solvent (B) can be appropriately selected within these ranges.

In a certain embodiment, the content of organic solvent (B) may be 5 mass% or more and less than 40 mass%, 8 mass% or more and less than 38 mass%, or 10 mass% or more and less than 35 mass%.

In another embodiment, the content of organic solvent (B) may be 35 mass% or more and 95 mass% or less, 38 mass% or more and 94 mass% or less, or 40 mass% or more and 92 mass% or less.

In view of even superior bond strength to stereolithographic articles and non-stereolithographic articles, the content of organic solvent (B) in a certain preferred embodiment may be 40 to 85 mass% of the total composition amount.

### [Polymerization Initiator (C)]

In view of improving curability, an adhesive composition for stereolithographic articles and non-stereolithographic articles of the present invention may further comprise a polymerization initiator (C). The polymerization initiator (C) used in the present invention may be selected from polymerization initiators used in industry, particularly those used in dentistry. The polymerization initiator (C) may be used alone, or two or more thereof may be used in combination. Specifically, the polymerization initiator (C) may be at least one selected from the group consisting of photopolymerization initiator (C-1) and chemical polymerization initiator (C-2).

Examples of the photopolymerization initiator (C-1) include (bis)acylphosphine oxides (including salts), thioxanthones (including salts such as quaternary ammonium salts), ketals, α-diketones, coumarins, anthraquinones, benzoinalkyl ether compounds, and α-aminoketone compounds.

Among these, the photopolymerization initiator (C-1) is preferably at least one selected from the group consisting of a (bis)acylphosphine oxide and an α-diketone. In this way, a curable composition can be provided that has excellent photocurability both in the visible light region and the near ultraviolet region, and that shows sufficient photocurability regardless of whether the light source used is a halogen lamp, a light emitting diode (LED), or a xenon lamp.

Examples of acylphosphine oxides in the (bis)acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylethoxyphenylphosphine oxide, 2,3,5,6-tetramethylbenzoyldiphenylphosphine oxide, benzoyl di(2,6-dimethylphenyl)phosphonate, sodium salts of 2,4,6-trimethylbenzoylphenylphosphine oxide, potassium salts of 2,4,6-trimethylbenzoylphenylphosphine oxide, and ammonium salts of 2,4,6-trimethylbenzoylphenylphosphine oxide.

Examples of bisacylphosphine oxides in the (bis)acylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, and bis(2,3,6-trimethylbenzoyl)-2,4,4-trimethylpentylphosphine oxide.

Examples of the (bis)acylphosphine oxides also include the compounds mentioned in JP 2000-159621 A.

Among these (bis)acylphosphine oxides, particularly preferred for use as photopolymerization initiator (C-1) are 2,4,6-trimethylbenzoyldiphenylphosphine oxide, 2,4,6-trimethylbenzoylmethoxyphenylphosphine oxide, and bis(2,4,6-trimethylbenzoyl)phenylphosphine oxide.

Examples of the α-diketones include diacetyl, benzyl, camphorquinone, 2,3-pentadione, 2,3-octadione, 9,10-phenanthrenequinone, 4,4'-oxybenzyl, and acenaphthenequinone. Particularly preferred is camphorquinone for its maximum absorption wavelength occurring in the visible light region.

Preferred for use as chemical polymerization initiator (C-2) are organic peroxides. The organic peroxides used as chemical polymerization initiator (C-2) are not particularly limited, and may be known organic peroxides. Typical examples of organic peroxides include ketone peroxides, hydroperoxides, diacyl peroxides, dialkyl peroxides, peroxyketals, peroxyesters, and peroxydicarbonates.

Examples of the ketone peroxides include methyl ethyl ketone peroxide, methyl isobutyl ketone peroxide, methyl cyclohexanone peroxide, and cyclohexanone peroxide.

Examples of the hydroperoxides include 2,5-dimethylhexane-2,5-dihydroperoxide, diisopropylbenzenehydroperoxide, cumenehydroperoxide, t-butyl hydroperoxide, and 1,1,3,3-tetramethylbutyl hydroperoxide.

Examples of the diacyl peroxides include acetyl peroxide, isobutyryl peroxide, benzoyl peroxide, decanoyl peroxide, 3,5,5-trimethylhexanoyl peroxide, 2,4-dichlorobenzoyl peroxide, and lauroyl peroxide.

Examples of the dialkyl peroxides include di-t-butyl peroxide, dicumyl peroxide, t-butyl cumyl peroxide, 2,5-dimethyl-2,5-di(t-butylperoxy)hexane, 1,3-bis(t-butylperoxyisopropyl)benzene, and 2,5-dimethyl-2,5-di(t-butylperoxy)-3-hexyne.

Examples of the peroxyketals include 1,1-bis(t-butylperoxy)-3,3,5-trimethylcyclohexane, 1,1-bis(t-butylperoxy)cyclohexane, 2,2-bis(t-butylperoxy)butane, 2,2-bis(t-butylperoxy)octane, n-butyl 4,4-bis(t-butylperoxy)valeric acid ester.

Examples of the peroxyesters include α-cumyl peroxyneodecanoate, t-butyl peroxyneodecanoate, t-butyl peroxypivalate, 2,2,4-trimethylpentylperoxy-2-ethylhexanoate, t-amylperoxy-2-ethylhexanoate, t-butyl peroxy-2-ethylhexanoate, di-t-butyl peroxyisophthalate, di-t-butyl peroxyhexahydroterephthalate, t-butyl peroxy-3,3,5-trimethylhexanoate, t-butyl peroxyacetate, t-butyl peroxybenzoate, and t-butyl peroxymaleate.

Examples of the peroxydicarbonates include di(3-methoxybutyl)peroxydicarbonate, di(2-ethylhexyl)peroxydicarbonate, bis(4-t-butylcyclohexyl)peroxydicarbonate, diisopropylperoxydicarbonate, di-n-propylperoxydicarbonate, di(2-ethoxyethyl)peroxydicarbonate, and diallyl peroxydicarbonate.

From an overall balance of safety, storage stability, and radical generating potential in the presence of polyfunctional acrylic polymerizable monomer (A), preferred for use as chemical polymerization initiator (C-2) among these organic peroxides are diacyl peroxides and hydroperoxides. Particularly preferred as chemical polymerization initiator (C-2) are benzoyl peroxide, cumene hydroperoxide, t-butyl hydroperoxide, and 1,1,3,3-tetramethylbutyl hydroperoxide.

The content of the polymerization initiator (C) in an adhesive composition for stereolithographic articles and non-stereolithographic articles of the present invention is not particularly limited. However, in view of considerations such as the curability of the adhesive composition for stereolithographic articles and non-stereolithographic articles obtained, the content of polymerization initiator (C) is preferably 0.001 to 30 parts by mass with respect to total 100 parts by mass of the polymerizable monomers. When the content of polymerization initiator (C) is 0.001 parts or more by mass, polymerization can sufficiently take place, preventing the occurrence of undesired stickiness. The content of polymerization initiator (C) is more preferably 0.01 parts or more by mass, even more preferably 0.1 parts or more by mass. When the content of polymerization initiator (C) is 30 parts or less by mass, the polymerization initiator (C) can be prevented from precipitating from the adhesive composition for stereolithographic articles and non-stereolithographic articles. The content of polymerization initiator (C) is more preferably 10 parts or less by mass, even more preferably 5.0 parts or less by mass, particularly preferably 1.0 part or less by mass.

### [Polymerization Accelerator (D)]

In view of improving curability, an adhesive composition for stereolithographic articles and non-stereolithographic articles of the present invention may further comprise a polymerization accelerator (D).

Examples of the polymerization accelerator (D) used in the present invention include amines, aldehydes, thiourea compounds, organic phosphorus compounds, borate compounds, barbituric acid compounds, triazine compounds, vanadium compounds, copper compounds, tin compounds, cobalt compounds, and halogen compounds. The polymerization accelerator (D) may be used alone, or two or more thereof may be used in combination.

The amines can be classified into aliphatic amines and aromatic amines. Examples of the aliphatic amines include primary aliphatic amines such as n-butylamine, n-hexylamine, and n-octylamine; secondary aliphatic amines such as diisopropylamine, dibutylamine, and N-methylethanolamine; and tertiary aliphatic amines such as N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine.

Examples of the aromatic amines include N,N-bis(2-hydroxyethyl)-3,5-dimethylaniline, N,N-bis(2-hydroxyethyl)-p-toluidine (hereinafter, also referred to by the abbreviation "DEPT"), N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, ethyl 4-(N,N-dimethylamino)benzoate, methyl 4-(N,N-dimethylamino)benzoate, n-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate.

In view of imparting excellent curability to an adhesive composition for stereolithographic articles and non-stereolithographic articles of the present invention, preferred for use is at least one selected from the group consisting of DEPT, ethyl 4-(N,N-dimethylamino)benzoate, n-butoxyethyl 4-(N,N-dimethylamino)benzoate, and 4-(N,N-dimethylamino)benzophenone.

Examples of the aldehydes include terephthalaldehyde, and derivatives of benzaldehyde. Examples of derivatives of benzaldehyde include dimethylaminobenzaldehyde, p-methoxybenzaldehyde, p-ethoxybenzaldehyde, and p-n-octyloxybenzaldehyde.

Examples of the thiourea compounds include 1-(2-pyridyl)-2-thiourea, thiourea, methylthiourea, ethylthiourea, N,N'-dimethylthiourea, N,N'-diethylthiourea, N,N'-di-n-propylthiourea, N,N'-dicyclohexylthiourea, trimethylthiourea, triethylthiourea, tri-n-propylthiourea, tricyclohexylthiourea, tetramethylthiourea, tetraethylthiourea, tetra-n-propylthiourea, tetracyclohexylthiourea, 3,3-dimethylethylenethiourea, and 4,4-dimethyl-2-imidazolidinethione. In view of improving curability in the presence of polyfunctional (meth)acrylic polymerizable monomer (A), preferred for use as polymerization accelerator (D) is 1-(2-pyridyl)-2-thiourea or 4,4-dimethyl-2-imidazolidinethione.

Examples of the organic phosphorus compounds include triphenylphosphine, 2-methyltriphenylphosphine, 4-methyltriphenylphosphine, 2-methoxytriphenylphosphine, 4-methoxytriphenylphosphine, tri-n-butylphosphine, triisobutylphosphine, and tri-t-butylphosphine.

Examples of the borate compounds include aryl borate compounds. Specific examples of arylborate compounds preferred for use include:
trialkylphenylboron, trialkyl(p-chlorophenyl)boron, trialkyl(p-fluorophenyl)boron, trialkyl[3,5-bis(trifluoromethyl)phenyl]boron, trialkyl[3,5-bis(1,1,1,3,3,3-hexafluoro-2-methoxy-2-propyl)phenyl]boron, trialkyl(p-nitrophenyl)boron, trialkyl(m-nitrophenyl)boron, trialkyl(p-butylphenyl)boron, trialkyl(m-butylphenyl)boron, trialkyl(p-butyloxyphenyl)boron, trialkyl(m-butyloxyphenyl)boron, trialkyl(p-octyloxyphenyl)boron, and trialkyl(m-octyloxyphenyl)boron (the alkyl group is at least one selected from the group consisting of, for example, an n-butyl group, an n-octyl group, and an n-dodecyl group), as well as salts of these (such as sodium salts, lithium salts, potassium salts, magnesium salts, tetrabutylammonium salts, tetramethylammonium salts, tetraethylammonium salts, methylpyridinium salts, ethylpyridinium salts, butylpyridinium salts, methylquinolinium salts, ethylquinolinium salts, and butylquinolinium salts);
dialkyldiphenylboron, dialkyldi(p-chlorophenyl)boron, dialkyldi(p-fluorophenyl)boron, dialkyldi[3,5-bis(trifluoromethyl)phenyl]boron, dialkyldi[3,5-bis(1,1,1,3,3,3-hexafluoro-2-methoxy-2-propyl)phenyl]boron, dialkyldi(p-nitrophenyl)boron, dialkyldi(m-nitrophenyl)boron, dialkyldi(p-butylphenyl)boron, dialkyldi(m-butylphenyl)boron, dialkyldi(p-butyloxyphenyl)boron, dialkyldi(m-butyloxyphenyl)boron, dialkyldi(p-octyloxyphenyl)boron, and dialkyldi(m-octyloxyphenyl)boron (the alkyl group is at least one selected from the group consisting of, for example, an n-butyl group, an n-octyl group, and an n-dodecyl group), as well as salts of these (such as sodium salts, lithium salts, potassium salts, magnesium salts, tetrabutylammonium salts, tetramethylammonium salts, tetraethylammonium salts, methylpyridinium salts, ethylpyridinium salts, butylpyridinium salts, methylquinolinium salts, ethylquinolinium salts, and butylquinolinium salts);
monoalkyltriphenylboron, monoalkyltri(p-chlorophenyl)boron, monoalkyltri(p-fluorophenyl)boron, monoalkyltri[3,5-bis(trifluoromethyl)phenyl]boron, monoalkyltri[3,5-bis(1,1,1,3,3,3-hexafluoro-2-methoxy-2-propyl)phenyl]boron, monoalkyltri(p-nitrophenyl)boron, monoalkyltri(m-nitrophenyl)boron, monoalkyltri(p-butylphenyl)boron, monoalkyltri(m-butylphenyl)boron, monoalkyltri(p-butyloxyphenyl)boron, monoalkyltri(m-butyloxyphenyl)boron, monoalkyltri(p-octyloxyphenyl)boron, and monoalkyltri(m-octyloxyphenyl)boron (the alkyl group is at least one selected from the group consisting of, for example, an n-butyl group, an n-octyl group, and an n-dodecyl group), as well as salts of these (such as sodium salts, lithium salts, potassium salts, magnesium salts, tetrabutylammonium salts, tetramethylammonium salts, tetraethylammonium salts, methylpyridinium salts, ethylpyridinium salts, butylpyridinium salts, methylquinolinium salts, ethylquinolinium salts, and butylquinolinium salts);
tetraphenylboron, tetrakis(p-chlorophenyl)boron, tetrakis(p-fluorophenyl)boron, tetrakis[3,5-bis(trifluoromethyl)phenyl]boron, tetrakis[3,5-bis(1,1, 1 ,3,3,3-hexafluoro-2-methoxy-2-propyl)phenyl]boron, tetrakis(p-nitrophenyl)boron, tetrakis(m-nitrophenyl)boron, tetrakis(p-butylphenyl)boron, tetrakis(m-butylphenyl)boron, tetrakis(p-butyloxyphenyl)boron, tetrakis(m-butyloxyphenyl)boron, tetrakis(p-octyloxyphenyl)boron, tetrakis(m-octyloxyphenyl)boron, (p-fluorophenyl)triphenylboron, [3,5-bis(trifluoromethyl)phenyl]triphenylboron, (p-nitrophenyl)triphenylboron, (m-butyloxyphenyl)triphenylboron, (p-butyloxyphenyl)triphenylboron, (m-octyloxyphenyl)triphenylboron, and (p-octyloxyphenyl)triphenylboron, as well as salts of these (such as sodium salts, lithium salts, potassium salts, magnesium salts, tetrabutylammonium salts, tetramethylammonium salts, tetraethylammonium salts, methylpyridinium salts, ethylpyridinium salts, butylpyridinium salts, methylquinolinium salts, ethylquinolinium salts, and butylquinolinium salts).

Examples of the barbituric acid compounds include barbituric acid, 1,3-dimethylbarbituric acid, 1,3-diphenylbarbituric acid, 1,5-dimethylbarbituric acid, 5-butylbarbituric acid, 5-ethylbarbituric acid, 5-isopropylbarbituric acid, 5-cyclohexylbarbituric acid, 1,3,5-trimethylbarbituric acid, 1,3-dimethyl-5-ethylbarbituric acid, 1,3-dimethyl-5-n-butylbarbituric acid, 1,3-dimethyl-5-isobutylbarbituric acid, 1,3-dimethyl-5-cyclopentylbarbituric acid, 1,3-dimethyl-5-cyclohexylbarbituric acid, 1,3-dimethyl-5-phenylbarbituric acid, 1-cyclohexyl-1-ethylbarbituric acid, 1-benzyl-5-phenylbarbituric acid, 5-methylbarbituric acid, 5-propylbarbituric acid, 1,5-diethylbarbituric acid, 1-ethyl-5-methylbarbituric acid, 1-ethyl-5-isobutylbarbituric acid, 1,3-diethyl-5-butylbarbituric acid, 1-cyclohexyl-5-methylbarbituric acid, 1-cyclohexyl-5-ethylbarbituric acid, 1-cyclohexyl-5-octylbarbituric acid, 1-cyclohexyl-5-hexylbarbituric acid, 5-butyl-1-cyclohexylbarbituric acid, and thiobarbituric acid, as well as salts of these (particularly preferred are alkali metal salts or alkali earth metal salts). Examples of the salts of barbituric acid include sodium 5-butylbarbiturate, sodium 1,3,5-trimethylbarbiturate, and sodium 1-cyclohexyl-5-ethylbarbiturate.

Examples of the triazine compounds include 2,4,6-tris(trichloromethyl)-s-triazine, 2,4,6-tris(tribromomethyl)-s-triazine, 2-methyl-4,6-bis(trichloromethyl)-s-triazine, 2-methyl-4,6-bis(tribromomethyl)-s-triazine, 2-phenyl-4,6-bis(trichloromethyl)-s-triazine, 2-(p-methoxyphenyl)-4,6-bis(trichloromethyl)-s-triazine, 2-(p-methylthiophenyl)-4,6-bis(trichloromethyl)-s-triazine, 2-(p-chlorophenyl)-4,6-bis(trichloromethyl)-s-triazine, 2-(2,4-dichlorophenyl)-4,6-bis(trichloromethyl)-s-triazine, 2-(p-bromophenyl)-4,6-bis(trichloromethyl)-s-triazine, 2-(p-tolyl)-4,6-bis(trichloromethyl)-s-triazine, 2-n-propyl-4,6-bis(trichloromethyl)-s-triazine, 2-(α,α,β-trichloroethyl)-4,6-bis(trichloromethyl)-s-triazine, 2-styryl-4,6-bis(trichloromethyl)-s-triazine, 2-[2-(p-methoxyphenyl)ethenyl]-4,6-bis(trichloromethyl)-s-triazine, 2-[2-(o-methoxyphenyl)ethenyl]-4,6-bis(trichloromethyl)-s-triazine, 2-[2-(p-butoxyphenyl)ethenyl]-4,6-bis(trichloromethyl)-s-triazine, 2-[2-(3,4-dimethoxyphenyl)ethenyl]-4,6-bis(trichloromethyl)-s-triazine, 2-[2-(3,4,5-trimethoxyphenyl)ethenyl]-4,6-bis(trichloromethyl)-s-triazine, 2-(1-naphthyl)-4,6-bis(trichloromethyl)-s-triazine, 2-(4-biphenylyl)-4,6-bis(trichloromethyl)-s-triazine, 2-[2-{N, N-bis(2-hydroxyethyl)am ino}ethoxy]-4,6-bis(trichloromethyl)-s-triazine, 2-[2-{N-hydroxyethyl-N-ethylam ino}ethoxy]-4,6-bis(trichloromethyl)-s-triazine, 2-[2-{N-hydroxyethyl-N-methylamino}ethoxy]-4,6-bis(trichloromethyl)-s-triazine, and 2-[2-{N, N-dial lylam ino}ethoxy]-4,6-bis(trichloromethyl)-s-triazine.

The vanadium compounds are preferably vanadium compounds with valences of IV and/or V. Examples of vanadium compounds with valences of IV and/or V include vanadium(IV) oxide, vanadyl(IV) acetylacetonate, vanadyl oxalate, vanadyl sulfate, vanadium(IV) oxobis(1-phenyl-1,3-butanedionate), bis(maltolato)oxovanadium(IV), vanadium(V) oxide, sodium metavanadate, and ammonium metavanadate. In view of the curability of the adhesive composition for stereolithographic articles and non-stereolithographic articles in the presence of polyfunctional (meth)acrylic polymerizable monomer (A), preferred for use as polymerization accelerator (D) is vanadyl(IV) acetylacetonate.

Examples of the copper compounds include copper acetylacetonate, copper(II) acetate, copper oleate, copper(II) chloride, and copper(II) bromide. In view of the curability of the adhesive composition for stereolithographic articles and non-stereolithographic articles in the presence of polyfunctional (meth)acrylic polymerizable monomer (A), preferred for use as polymerization accelerator (D) is copper acetylacetonate or copper(II) acetate.

Examples of the tin compounds include di-n-butyltin dimaleate, di-n-octyltin dimaleate, di-n-octyltin dilaurate, and di-n-butyltin dilaurate.

Examples of the cobalt compounds include acetylacetone cobalt, cobalt acetate, cobalt oleate, cobalt chloride, and cobalt bromide.

Preferred for use as halogen compounds are, for example, dilauryldimethylammonium chloride, lauryldimethylbenzylammonium chloride, benzyltrimethylammonium chloride, tetramethylammonium chloride, benzyldimethylcetylammonium chloride, and dilauryldimethylammonium bromide.

Particularly preferred for use among these polymerization accelerators (D) are amines, thiourea compounds, vanadium compounds, and copper compounds. Most preferred for use as polymerization accelerator (D) are 1-(2-pyridyl)-2-thiourea, ethyl 4-(N,N-dimethylamino)benzoate, 4,4-dimethyl-2-imidazolidinethione, vanadyl(IV) acetylacetonate, copper acetylacetonate, and copper(II) acetate.

The content of the polymerization accelerator (D) in a curable composition of the present invention is not particularly limited. However, in view of considerations such as the curability of the adhesive composition for stereolithographic articles and non-stereolithographic articles obtained. The content of polymerization accelerator (D) is preferably 0.001 to 30 parts by mass with respect to total 100 parts by mass of the polymerizable monomers. When the content of polymerization accelerator (D) is 0.001 parts or more by mass, polymerization can sufficiently take place, preventing the occurrence of undesired stickiness. The content of polymerization accelerator (D) is more preferably 0.01 parts or more by mass, even more preferably 0.1 parts or more by mass. When the content of polymerization accelerator (D) is 30 parts or less by mass, the polymerization accelerator (D) can be prevented from precipitating from the adhesive composition for stereolithographic articles and non-stereolithographic articles. The content of polymerization accelerator (D) is more preferably 10 parts or less by mass, even more preferably 5.0 parts or less by mass.

An adhesive composition for stereolithographic articles and non-stereolithographic articles of the present invention is not particularly limited, as long as it comprises the polyfunctional (meth)acrylic polymerizable monomer (A) and organic solvent (B). However, in view of exhibiting superior adhesive properties to both stereolithographic articles and non-stereolithographic articles, it is preferable that an adhesive composition for stereolithographic articles and non-stereolithographic articles of the present invention be essentially free of polymer components other than polyfunctional (meth)acrylic polymerizable monomer (A).

Here, "polymer component" refers to polymers with an weight-average molecular weight of 1,000 or more.

Such polymer components other than polyfunctional (meth)acrylic polymerizable monomer (A) are not particularly limited, and examples include acrylic polymers, silicone polymers (such as silicone rubber, and polyvinyl silicone), polyolefin polymers, fluoro polymers, (meth)acrylic polymers ((meth)acryloxy group-containing polymers), and (meth)acrylamide polymers ((meth)acrylamide group-containing polymers).

The phrase "essentially free of polymer components" means that the content of polymer components other than polyfunctional (meth)acrylic polymerizable monomer (A) in the adhesive composition for stereolithographic articles and non-stereolithographic articles is 1 mass% or less. Similarly, the phrase "essentially free of oligomer components", described later, means that the content of oligomer components is 1 mass% or less, as with the case of "essentially free of polymer components".

A certain embodiment is, for example, an adhesive composition for stereolithographic articles and non-stereolithographic articles comprising 1 mass% or less of polymer components with a weight-average molecular weight of 1,000 or more. When the content of such polymer components in the adhesive composition for stereolithographic articles and non-stereolithographic articles is more than 1 mass%, it tends to lead to a decrease in adhesive properties to stereolithographic articles. In the present embodiment, the content of such polymer components in the adhesive composition for stereolithographic articles and non-stereolithographic articles is preferably 0.5 mass% or less, more preferably 0.1 mass% or less, even more preferably 0.05 mass% or less.

A certain preferred embodiment is, for example, an adhesive composition for stereolithographic articles and non-stereolithographic articles that comprises a polyfunctional (meth)acrylic polymerizable monomer (A) and an organic solvent (B), and that is essentially free of polymer components and oligomer components.

Another embodiment is, for example, an adhesive composition for stereolithographic articles and non-stereolithographic articles in which the content of polymer components is less than 0.1 parts by mass relative to 100 parts by mass of organic solvent (B). In the present embodiment, the term "polymer component" can be read as referring to "oligomer component", or "polymer component and oligomer component".

An adhesive composition for stereolithographic articles and non-stereolithographic articles of the present invention may comprise components other than the polyfunctional (meth)acrylic polymerizable monomer (A), organic solvent (B), polymerization initiator (C), and polymerization accelerator (D). An adhesive composition for stereolithographic articles and non-stereolithographic articles of the present invention can be produced following known methods.

An adhesive composition for stereolithographic articles and non-stereolithographic articles of the present invention may additionally comprise a filler to adjust its paste properties. Examples of such fillers include inorganic fillers, and organic-inorganic composite fillers. The filler may be used alone, or two or more thereof may be used in combination.

Examples of inorganic filler materials include quartz, silica, alumina, silica-titania, silica-titania-barium oxide, silica-zirconia, silica-alumina, lanthanum glass, borosilicate glass, soda glass, barium glass, strontium glass, glass-ceramic, aluminosilicate glass, barium boroaluminosilicate glass, strontium boroaluminosilicate glass, fluoroaluminosilicate glass, calcium fluoroaluminosilicate glass, strontium fluoroaluminosilicate glass, barium fluoroaluminosilicate glass, and strontium calcium fluoroaluminosilicate glass. These may be used alone, or two or more thereof may be used in combination. The inorganic fillers are not limited to particular shapes, and may be appropriately selected from fillers of various shapes, such as irregularly shaped fillers and spherical fillers.

Examples of the organic-inorganic composite fillers include those with an inorganic filler dispersed in an organic filler, and organic-inorganic composite fillers where the inorganic filler is coated with various polymerizable monomers.

An adhesive composition for stereolithographic articles and non-stereolithographic articles of the present invention may also comprise known stabilizers to reduce deterioration or adjust photocurability. Examples of such stabilizers include polymerization inhibitors, ultraviolet absorbers, and antioxidants. The stabilizer may be used alone, or two or more thereof may be used in combination.

Examples of the polymerization inhibitors include hydroquinone, hydroquinone monomethyl ether, dibutylhydroquinone, dibutylhydroquinone monomethyl ether, 4-t-butylcatechol, 2-t-butyl-4,6-dimethylphenol, 2,6-di-t-butylphenol, and 3,5-di-t-butyl-4-hydroxytoluene. The content of polymerization inhibitors is preferably 0.001 to 5.0 parts by mass with respect to total 100 parts by mass of the polymerizable monomers.

An adhesive composition for stereolithographic articles and non-stereolithographic articles of the present invention may also comprise known additives to adjust shade or paste properties. Examples of such additives include pigments, dyes, thickeners, and aryl compounds (for example, aryliodonium salts). The additives may be used alone, or two or more thereof may be used in combination.

An adhesive composition for stereolithographic articles and non-stereolithographic articles of the present invention exhibits excellent adhesive properties to both stereolithographic articles, and non-stereolithographic articles polymerized by methods other than stereolithography. Stereolithography usually involves secondary polymerization to polymerize polymerizable monomers that remain unreacted after shaping. A feature of an adhesive composition for stereolithographic articles and non-stereolithographic articles of the present invention is that it exhibits excellent adhesive properties even when the monomers have hardened through polymerization after considerable time has passed since this secondary polymerization and shaping process.

An adhesive composition for stereolithographic articles and non-stereolithographic articles of the present invention can be used in applications where this feature is beneficial. An adhesive composition for stereolithographic articles and non-stereolithographic articles of the present invention is particularly suited for lining and repairing denture base materials, and mouthpiece-like appliances used for treating sleep disorders. For lining of denture base materials, an adhesive composition for stereolithographic articles and non-stereolithographic articles of the present invention can be used not only as a bonding agent in traditional lining methods but also as a denture base lining material itself.

A certain embodiment is, for example, a bonding agent for dental oral appliances that comprises any of the adhesive compositions for stereolithographic articles and non-stereolithographic articles above.

Another embodiment is, for example, a bonding agent for denture bases that comprises any of the adhesive compositions for stereolithographic articles and non-stereolithographic articles above.

Yet another embodiment is, for example, a bonding agent for dental occlusal splints that comprises any of the adhesive compositions for stereolithographic articles and non-stereolithographic articles above.

Still another embodiment is, for example, a bonding agent for therapeutic appliances for sleep apnea that comprises any of the adhesive compositions for stereolithographic articles and non-stereolithographic articles above.

A certain embodiment is, for example, a method for lining or repairing stereolithographic articles and non-stereolithographic articles fabricated by stereolithography or methods other than stereolithography, using any of the adhesive compositions for stereolithographic articles and non-stereolithographic articles above.

The method for lining or repairing stereolithographic articles and non-stereolithographic articles may employ known techniques, and is not particularly limited. Examples of methods other than stereolithography include thermal polymerization and photopolymerization. The heating temperature in thermal polymerization is not particularly limited, as long as it allows a polymerization reaction to proceed. The photoirradiation in photopolymerization is not particularly limited, and known photoirradiators may be used, as long as it allows a polymerization reaction to proceed.

An adhesive composition for stereolithographic articles and non-stereolithographic articles of the present invention can be applied to stereolithographic articles such as denture base materials, dental mouthpieces, and appliances for treating sleep apnea.

The stereolithographic articles are not particularly limited, and may be, for example, articles stereolithographically created from a composition that comprises a polymerizable monomer and a photopolymerization initiator, and in which the polymerizable monomer comprises at least one selected from the group consisting of a urethanized (meth)acrylic acid ester compound (a-1), and an aromatic (meth)acrylic acid ester compound (a-2) with no urethane bond.

The urethanized (meth)acrylic acid ester compound (a-1), and the aromatic (meth)acrylic acid ester compound (a-2) with no urethane bond may be the compounds described in conjunction with the adhesive composition for stereolithographic articles and non-stereolithographic articles. The photopolymerization initiator may be the photopolymerization initiator (C-1) described in conjunction with the adhesive composition for stereolithographic articles and non-stereolithographic articles.

In certain embodiments, the stereolithographic articles may be, for example, articles stereolithographically created from a composition that comprises a polymerizable monomer and a photopolymerization initiator, and in which the polymerizable monomer comprises a urethanized (meth)acrylic acid ester compound (a-1).

An advantage of an adhesive composition for stereolithographic articles and non-stereolithographic articles of the present invention is that it can be used for both bonding of stereolithographic articles and bonding of non-stereolithographic articles. However, an adhesive composition for stereolithographic articles and non-stereolithographic articles of the present invention may be provided as an adhesive composition for stereolithographic articles.

Another embodiment is, for example, use of the adhesive composition to bond stereolithographic articles.

An adhesive composition for stereolithographic articles and non-stereolithographic articles of the present invention is applicable to non-stereolithographic articles such as denture base materials, dental mouthpieces, appliances for treating sleep apnea, and dental restoration materials.

Examples of non-stereolithographic articles to which an adhesive composition for stereolithographic articles and non-stereolithographic articles of the present invention is applicable include acrylic resins (preferably, polymethyl methacrylate (PMMA)), and thermoplastic elastomers (for example, elastomers containing 50 mass% or more of PMMA, along with a styrene-isoprene-styrene block copolymer).

Preferred examples of non-stereolithographic articles to which the composition is applicable include non-stereolithographic articles containing 50 mass% or more of PMMA.

Another embodiment is, for example, use of the adhesive composition to bond non-stereolithographic articles.

In an adhesive composition for stereolithographic articles and non-stereolithographic articles of the present invention, the type and content of polyfunctional (meth)acrylic polymerizable monomer (A), organic solvent (B), and various optional components (such as polymerization initiators, polymerization accelerators, fillers, stabilizers, and additives) may be adjusted, as required, to adjust the coating thickness or adhesive properties in lining or repairing denture base materials, and mouthpiece-like appliances used for treating sleep disorders.

Another certain embodiment is, for example, an adhesive composition comprising:
a polyfunctional (meth)acrylic polymerizable monomer (A); and
an organic solvent (B) having a normal boiling point of 120°C or less, and a viscosity at 25°C of 10 mPa·s or less,
wherein the polyfunctional (meth)acrylic polymerizable monomer (A) comprises two or more polymerizable groups per molecule, and at least one selected from the group consisting of a urethane bond, an aromatic skeleton, and an amide bond,
the polymerizable groups of the polyfunctional (meth)acrylic polymerizable monomer (A) are (meth)acryloxy groups or (meth)acrylamide groups, and
the polyfunctional (meth)acrylic polymerizable monomer (A) comprises a urethanized (meth)acrylic acid ester compound (a-1).

The adhesive compositions of such embodiments also exhibit excellent adhesive properties to a variety of stereolithographic articles and non-stereolithographic articles.

The adhesive compositions may be adhesive compositions for intraoral use, or may be dental adhesive compositions. The adhesive compositions for intraoral use are not particularly limited, as long as it is intended for human oral use. Examples include therapeutic applications in dentistry, oral surgery, respiratory medicine, and cardiovascular medicine, as well as sports applications.

The present invention encompasses embodiments combining all or part of the foregoing features in various ways within the technical idea of the present invention, provided that the present invention can exhibit its effects (for example, "adhesive composition for stereolithographic articles and non-stereolithographic articles" may be modified to "adhesive composition", "adhesive composition for intraoral use", or "dental adhesive composition").

For example, various modifications can be made to all or part of the embodiments of "adhesive composition", based on the descriptions concerning "adhesive composition for stereolithographic articles and non-stereolithographic articles".

### EXAMPLES

The following describes the present invention in greater detail by way of Examples. It should be noted, however, that the present invention is in no way limited by the following Examples, and various changes may be made by a person with ordinary skill in the art within the technical idea of the present invention.

The components used for the adhesive compositions for stereolithographic articles and non-stereolithographic articles according to Examples and Comparative Examples are described below, along with the abbreviations.

### [Polyfunctional (meth)acrylic polymerizable monomer (A)]

### [Urethanized (meth)acrylic acid ester compound (a-1)]

UDMA: 2,2,4-trimethylhexamethylenebis(2-carbamoyloxyethyl)dimethacrylate (manufactured by Kyoeisha Chemical Co., Ltd.)
UA-122P: polyester-based urethane diacrylate (manufactured by Shin-Nakamura Chemical Co., Ltd.; Mw: 1,100, the number of polymerizable groups: 2, containing phenoxydiethylene glycol acrylate)

### [Aromatic (meth)acrylic acid ester compound (a-2) with no urethane bond]

Bis-GMA: 2,2-bis[4-(2-hydroxy-3-methacryloyloxypropoxy)phenyl]propane (manufactured by Shin-Nakamura Chemical Co., Ltd.) [Polymerizable monomer (a-3) containing (meth)acrylamide]
MAEA: 2-methacryloyloxyethylacrylamide (manufactured by KJ Chemicals Corporation)

### [Organic solvent (B)]

Ethyl acetate (normal boiling point: 77°C, viscosity (25°C): 0.44 mPa s, manufactured by Fujifilm Wako Pure Chemical Industries, Ltd.)
Methylene chloride (normal boiling point: 40°C, viscosity (25°C): 0.43 mPas, manufactured by Fujifilm Wako Pure Chemical Industries, Ltd.)
MMA: methyl methacrylate (normal boiling point: 100°C, viscosity (25°C): 0.56 mPas, manufactured by Fujifilm Wako Pure Chemical Industries, Ltd.)

### [Photopolymerization initiator (C-1)]

TPO: 2,4,6-trimethylbenzoyldiphenylphosphine oxide

### [Chemical polymerization initiator (C-2)]

BPO: benzoyl peroxide

### [Polymerization accelerator (D)]

DEPT: N,N-bis(2-hydroxyethyl)-p-toluidine

### [Polyfunctional (meth)acrylic polymerizable monomers other than polyfunctional (meth)acrylic polymerizable monomer (A)]

BMHPE: 1,2-bis(3-methacryloyloxy-2-hydroxypropoxy)ethane (manufactured by Shin-Nakamura Chemical Co., Ltd.)

### [Polymerizable monomers used in Comparative Examples]

ACMO: N-acryloylmorpholine (manufactured by KJ Chemicals Corporation)
HEMA: 2-hydroxyethyl methacrylate (manufactured by Kyoeisha Chemical Co., Ltd.)
MDP: 10-methacryloyloxydecyl hydrogen phosphate (manufactured by Kuraray Noritake Dental Inc.)
4-META: 4-methacryloyloxyethyl trimellitate anhydride (manufactured by Fujifilm Wako Pure Chemical Industries, Ltd.)
MPS: 3-methacryloyloxypropyl trimethoxysilane (manufactured by Shin-Etsu Chemical Co., Ltd.)
TMPT: trimethylolpropane trimethacrylate (manufactured by Kyoeisha Chemical Co., Ltd.)
PEMA: polyethyl methacrylate (manufactured by Negami Chemical Industrial Co., Ltd., Mw: 400,000)

Note that the weight-average molecular weight (Mw) means a weight-average molecular weight in terms of polystyrene as determined by gel permeation chromatography (GPC).

### <Reference Example 1>

### [Production of Photocurable Resin Composition 1]

A 2-L brown-colored wide-neck polyethylene bottle was charged with 700 g of 2,2,4-trimethylhexamethylenebis(2-carbamoyloxyethyl)dimethacrylate, 300 g of m-phenoxybenzyl acrylate, 30 g of 2,4,6-trimethylbenzoyldiphenylphosphine oxide, and 5.0 g of BHT (dibutylhydroxytoluene). These were then stirred at 25°C for 48 hours with a mechanical stirrer inserted into the bottle. After confirming that the components had fully dissolved, the resulting composition was designated as photocurable resin composition 1.

### <Reference Example 2>

### [Production of Photocurable Resin Composition 2]

A 2-L brown-colored wide-neck polyethylene bottle was charged with 600 g of ethoxylated bisphenol-A diacrylate (ABE-300 manufactured by Shin-Nakamura Chemical Co., Ltd.), 400 g of m-phenoxybenzyl acrylate, 30 g of 2,4,6-trimethylbenzoyldiphenylphosphine oxide, and 5.0 g of BHT These were then stirred at 25°C for 48 hours with a mechanical stirrer inserted into the bottle. After confirming that the components had fully dissolved, the resulting composition was designated as photocurable resin composition 2.

### [Examples 1 to 15 and Comparative Examples 1 to 11]

The components were mixed at ordinary temperature (20°C ± 15°C, JIS (Japanese Industrial Standards) Z 8703:1983) in the quantities indicated in Tables 1 and 2 to prepare pastes that served as adhesive compositions for stereolithographic articles and non-stereolithographic articles according to Examples 1 to 15 and Comparative Examples 1 to 11.

### <Adhesive Properties to Stereolithographic Article>

Using the photocurable resin compositions 1 and 2 obtained in Reference Examples, test specimens measuring 20 mm in diameter and 15 mm in height were fabricated with a stereolithography machine (DIGITALWAX^{®} 020D manufactured by DWS). These specimens underwent 2,000 flashes with a photoirradiator (Otoflash^{®} G171 manufactured by EnvisionTEC) to complete the curing process. After a two-week period, one flat surface of each specimen was polished under running water using #600 silicon carbide paper (manufactured by Nihon Kenshi Co., Ltd.), and the surface water was blown off with a dental air syringe, resulting in the creation of stereolithographic articles as stereolithographic adherends.

The adhesive composition for stereolithographic articles and non-stereolithographic articles of each Example and Comparative Example was applied to the polished surface of each stereolithographic adherend using a brush. The applied composition was thinly spread using a dental air syringe. Subsequently, Examples 2 and 5 and Comparative Example 2 (containing TPO) were subjected to 1,000 flashes using an irradiator (Otoflash^{®} G171 manufactured by EnvisionTEC). Examples 1, 3, 4, and 6 to 15, and Comparative Examples 1, and 3 to 11 (containing no TPO) did not undergo flash irradiation. Thereafter, a silicon sheet, approximately 1.0 mm thick with a 4 mm round hole, was attached to define the bonding area.

Following this, except for Comparative Example 1, the round hole was filled with a denture base lining material (manufactured by Kuraray Noritake Dental Inc. under the trade name Kuraribase^{®}), without applying the designated bonding agent. After attaching a 1 cm × 1 cm PET film under applied pressure, a 500 g weight was placed thereon via a glass plate (1 cm × 1 cm, 5.0 mm thick). With the weight placed on top, the material was left at room temperature for 1 hour to cure. In Comparative Example 1, Kuraribase was filled after applying the bonding agent designated for Kuraribase and removing the solvent with a dental air syringe, without photoirradiation. The subsequent curing process followed the same procedure used in the other Examples.

Subsequently, the weight and PET film were removed, and the remaining structure was immersed in distilled water. After storing it for 24 hours in a thermostatic chamber maintained at 37°C, a shear adhesion test was conducted at a crosshead speed of 1 mm/min, using a universal testing machine (Autograph AG-I 100kN, manufactured by Shimadzu Corporation). A total of five adhesion test samples were prepared, and the mean value was calculated for these samples. In this test, a bond strength of 5 MPa or higher indicates that the adhesive composition is usable in clinical applications. The bond strength was considered favorable when it was 7.5 MPa or higher, and excellent when it was 10 MPa or higher.

### <Adhesive Properties to Acrylic Resin Molded Article (Non-Stereolithographic Article)>

The heat-polymerizing denture base resin (manufactured by GC under the trade name Acron^{®}; containing polymethyl methacrylate (PMMA) powder and methyl methacrylate liquid as main components) was mixed following the instructions provided in the product. The mixture was then filled into a cylindrical SUS ring (measuring 20 mm in inner diameter and 15 mm in height) placed on a glass slide with a 50 µm thick PE film covering the glass slide. After placing a 50 µm thick PET film and another glass side on top, these were secured with clips. Subsequently, the resin was cured through thermal polymerization following the manufacturer's instructions. After two weeks, one flat surface was polished under running water with #600 silicon carbide paper (manufactured by Nihon Kenshi Co., Ltd.), and the surface water was blown off with a dental air syringe to obtain an adherend.

In each Example and Comparative Example, the non-stereolithographic adherend was evaluated for its adhesive properties, instead using the stereolithographic adherends evaluated in the foregoing section <Adhesive Properties to Stereolithographic Article>.

In each Example and Comparative Example, the evaluation was carried out following the same method described in <Adhesive Properties to Stereolithographic Article>, except for changing the type of adherend.

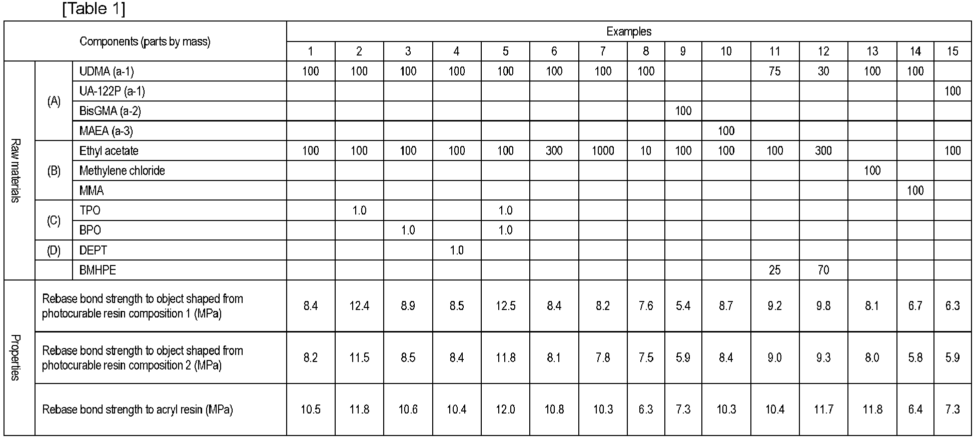

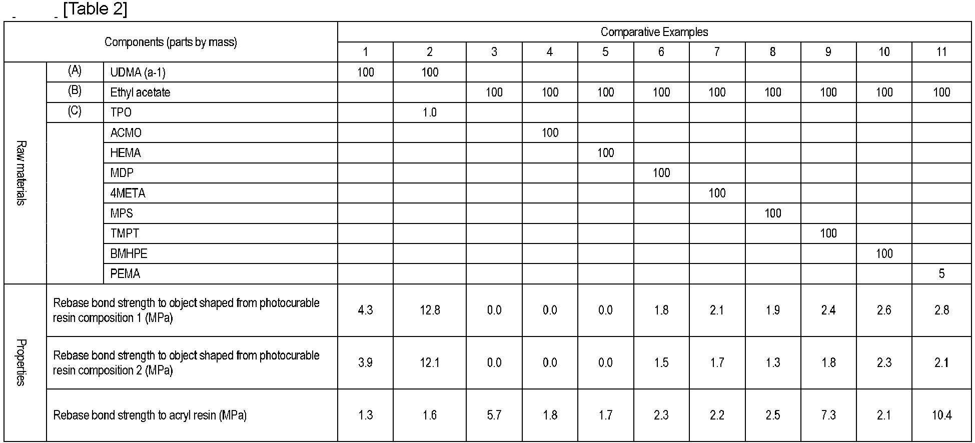

As indicated in Tables 1 and 2, the adhesive compositions for stereolithographic articles and non-stereolithographic articles according to Examples 1 to 15 demonstrated superior bond strength to stereolithographic articles compared to the compositions of Comparative Examples 1, and 3 to 11 lacking polyfunctional (meth)acrylic polymerizable monomer (A) or organic solvent (B).

The adhesive compositions for stereolithographic articles and non-stereolithographic articles according to Examples 1 to 15 also exhibited superior bond strength to acrylic resin compared to the compositions of Comparative Examples 1 to 8, and 10 lacking polyfunctional (meth)acrylic polymerizable monomer (A) or organic solvent (B).

### INDUSTRIAL APPLICABILITY

An adhesive composition for stereolithographic articles and non-stereolithographic articles of the present invention with adhesive properties exhibits excellent adhesive properties to both stereolithographic articles and non-stereolithographic articles. This enables the repairing or lining of not only stereolithographic articles but also non-stereolithographic articles fabricated from acrylic resins such as PMMA using non-stereolithography methods.

An adhesive composition for stereolithographic articles and non-stereolithographic articles of the present invention with adhesive properties is particularly suited for lining or repairing denture base materials, dental occlusal splints, and appliances used for treating sleep disorders.

Specifically, in dental treatment, it is no longer necessary to determine whether the denture base material is a stereolithographic article or non-stereolithographic article in lining denture bases, enabling use regardless of the denture base material.

Traditional adhesive compositions (for example, those primarily composed of (meth)acrylate polymers) may lack adhesive properties depending on the denture base material, necessitating a process to assess the denture base material. If the denture base material cannot be visually distinguished with certainty, there remains a risk of detachment when repairing or lining the denture base.

In contrast to such traditional adhesive compositions, an adhesive composition of the present invention possesses excellent adhesive properties to both stereolithographic articles and non-stereolithographic articles, regardless of the denture base material. This eliminates the need to assess the denture base material, and removes the risk of detachment, offering significant advantages in clinical dental practice.

## Claims

1. An adhesive composition for stereolithographic articles and non-stereolithographic articles, comprising:
a polyfunctional (meth)acrylic polymerizable monomer (A); and
an organic solvent (B) having a normal boiling point of 120°C or less, and a viscosity at 25°C of 10 mPa·s or less,
wherein the polyfunctional (meth)acrylic polymerizable monomer (A) comprises two or more polymerizable groups per molecule, and at least one selected from the group consisting of a urethane bond, an aromatic skeleton, and an amide bond, and
the polymerizable groups of the polyfunctional (meth)acrylic polymerizable monomer (A) are (meth)acryloxy groups or (meth)acrylamide groups.

2. The adhesive composition for stereolithographic articles and non-stereolithographic articles according to claim 1, wherein the polyfunctional (meth)acrylic polymerizable monomer (A) comprises at least one selected from the group consisting of a urethanized (meth)acrylic acid ester compound (a-1), an aromatic (meth)acrylic acid ester compound (a-2) with no urethane bond, and a polymerizable monomer (a-3) containing (meth)acrylamide.

3. The adhesive composition for stereolithographic articles and non-stereolithographic articles according to claim 2, wherein the polyfunctional (meth)acrylic polymerizable monomer (A) comprises the urethanized (meth)acrylic acid ester compound (a-1).

4. The adhesive composition for stereolithographic articles and non-stereolithographic articles according to claim 2, wherein the polyfunctional (meth)acrylic polymerizable monomer (A) comprises the urethanized (meth)acrylic acid ester compound (a-1), and the urethanized (meth)acrylic acid ester compound (a-1) is a compound with no polymer structure.

5. The adhesive composition for stereolithographic articles and non-stereolithographic articles according to claim 4, wherein the urethanized (meth)acrylic acid ester compound (a-1) comprises at least one selected from the group consisting of 2,2,4-trimethylhexamethylenebis(2-carbamoyloxyethyl)dimethacrylate, and N,N'-(2,2,4-trimethylhexamethylene)bis[2-(aminocarboxy)propane-1,3-diol]tetramethacry late.

6. The adhesive composition for stereolithographic articles and non-stereolithographic articles according to claim 2, wherein the polyfunctional (meth)acrylic polymerizable monomer (A) comprises the aromatic (meth)acrylic acid ester compound (a-2) with no urethane bond, and the aromatic (meth)acrylic acid ester compound (a-2) with no urethane bond further comprises a hydroxyl group.

7. The adhesive composition for stereolithographic articles and non-stereolithographic articles according to claim 2, wherein the polyfunctional (meth)acrylic polymerizable monomer (A) comprises the polymerizable monomer (a-3) containing (meth)acrylamide, and the polymerizable monomer (a-3) containing (meth)acrylamide comprises at least one selected from the group consisting of a primary amide bond and a secondary amide bond.

8. The adhesive composition for stereolithographic articles and non-stereolithographic articles according to any one of claims 1 to 7, wherein the organic solvent (B) comprises at least one selected from the group consisting of a halogenated hydrocarbon solvent, an ether solvent, an ester solvent, a ketone solvent, a non-aromatic hydrocarbon solvent, and an alcohol solvent.

9. The adhesive composition for stereolithographic articles and non-stereolithographic articles according to any one of claims 1 to 8, wherein the organic solvent (B) comprises at least one selected from the group consisting of ethyl acetate, methylene chloride, and acetone.

10. The adhesive composition for stereolithographic articles and non-stereolithographic articles according to any one of claims 1 to 9, wherein the content of the organic solvent (B) is 40 to 85 mass%.

11. The adhesive composition for stereolithographic articles and non-stereolithographic articles according to any one of claims 1 to 10, which comprises 1 mass% or less of a polymer component with a weight-average molecular weight of 1,000 or more.

12. The adhesive composition for stereolithographic articles and non-stereolithographic articles according to any one of claims 1 to 11, which further comprises a polymerization initiator (C).

13. The adhesive composition for stereolithographic articles and non-stereolithographic articles according to any one of claims 1 to 12, which further comprises a polymerization accelerator (D).

14. A bonding agent for dental oral appliances, comprising an adhesive composition for stereolithographic articles and non-stereolithographic articles of any one of claims 1 to 13.

15. A bonding agent for denture bases, comprising an adhesive composition for stereolithographic articles and non-stereolithographic articles of any one of claims 1 to 13.

16. A bonding agent for dental occlusal splints, comprising an adhesive composition for stereolithographic articles and non-stereolithographic articles of any one of claims 1 to 13.

17. A bonding agent for therapeutic appliances for sleep apnea, comprising an adhesive composition for stereolithographic articles and non-stereolithographic articles of any one of claims 1 to 13.

18. A method for lining or repairing stereolithographic articles and non-stereolithographic articles fabricated by stereolithography or methods other than stereolithography, using an adhesive composition for stereolithographic articles and non-stereolithographic articles of any one of claims 1 to 13.

19. An adhesive composition comprising:
a polyfunctional (meth)acrylic polymerizable monomer (A); and
an organic solvent (B) having a normal boiling point of 120°C or less, and a viscosity at 25°C of 10 mPa·s or less,
wherein the polyfunctional (meth)acrylic polymerizable monomer (A) comprises two or more polymerizable groups per molecule, and at least one selected from the group consisting of a urethane bond, an aromatic skeleton, and an amide bond,
the polymerizable groups of the polyfunctional (meth)acrylic polymerizable monomer (A) are (meth)acryloxy groups or (meth)acrylamide groups, and
the polyfunctional (meth)acrylic polymerizable monomer (A) comprises a urethanized (meth)acrylic acid ester compound (a-1).
